# EUROPEAN PATENT APPLICATION

(11) **EP 4 748 445 A2**
(43) Date of publication of application: **27.05.2026**
(21) Application number: 26170710.3
(22) Date of filing: 24.12.2021
(51) Int. Cl.: A61P 29/00

(54) **BORATE DERIVATIVE AND USES THEREOF**

(30) Priority: 25.12.2020 CN 202011558776; 01.04.2021 CN 202110356037; 26.11.2021 CN 202111419717
(62) Divisional of application: 21909545.2
(71) Applicant: Reistone Biopharma Company Limited, Shanghai 201210 (CN)
(72) Inventor: WANG, Zhongli, Shanghai, 201210 (CN); HAO, Xin, Shanghai, 201210 (CN); LIU, Sang, Shanghai, 201210 (CN)
(74) Representative: Cabinet Beau de Loménie

(57) **Abstract**

Provided are a compound as represented by formula I or a pharmaceutically acceptable salt thereof, and applications of the compound in preparing a medicament for preventing and/or treating a PDE-related disorder.

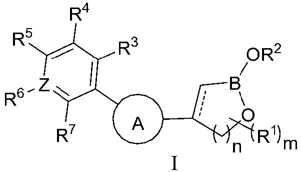

## Description

### FIELD OF THE INVENTION

The present disclosure belongs to the field of medicine and relates to a borate derivative and uses thereof.

### BACKGROUND OF THE INVENTION

Phosphodiesterases (PDEs) are a class of intracellular enzymes that cleavage phosphodiester bonds on the second messenger molecules 3',5'-cyclic adenosine monophosphate (cAMP) and 3',5'-cyclic guanosine monophosphate (cGMP). The cyclic nucleotides cAMP and cGMP act as second messengers in various cellular pathways. Among them, PDE4 is highly specific to cAMP and has four subtypes: PDE4A, PDE4B, PDE4C and PDE4D. PDE4 is involved in promoting monocyte and macrophage activation, neutrophil infiltration, vascular smooth muscle proliferation, vasodilation and myocardial contraction and other related physiological and pathological processes, and has effects on central nervous system function, cardiovascular function, inflammation/immune system, cell adhesion, and the like. PDE4 plays a major regulatory role in the expression of pro-inflammatory and anti-inflammatory mediators, and PDE4 inhibitors can inhibit the release of harmful mediators by inflammatory cells.

Many PDE4 inhibitors have been identified in recent years. For example, roflumilast is approved for severe chronic obstructive pulmonary disease (COPD) to reduce the times of sudden onset or to prevent worsening of COPD symptoms, and apremilast is approved for the treatment of adults with active psoriasis arthritis. Although PDE4 inhibitors show good pharmacological activity, these PDE inhibitors have side effects, such as induced gastrointestinal symptoms such as emesis and diarrhea. There is still a need to develop selective PDE4 inhibitors, especially selective PDE4 inhibitors with affinity to PDE4B and PDE4D.

The boron (B)-containing drug, Crisaborole was approved by FDA on December 14, 2016, as a topical treatment for mild to moderate atopic dermatitis. The boron atom facilitates skin penetration and binds to the bimetallic center of Phosphodiesterase 4 (PDE4). In addition, other boron-containing PDE inhibitor small molecules have been reported, such as CN102014927A, WO2020070651. However, the compounds of the present disclosure are not disclosed in any literature, and such compounds exhibit specific PDE4 inhibition effects.

### SUMMARY OF THE INVENTION

The present disclosure provides a compound of formula I or a pharmaceutically acceptable salt thereof, 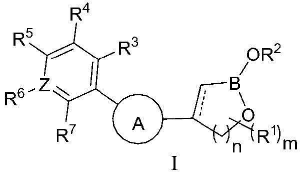
wherein, ring A is selected from the group consisting of 5-6 membered aryl ring and heteroaryl ring, the aryl ring or heteroaryl ring is optionally further substituted with one or more of R^{A1};
R^{A1} is selected from the group consisting of halogen, deuterium, hydroxy, nitro, cyano, amino, C₁₋₆ alkyl, C₃₋₆ cycloalkyl, 3-6 membered heterocyclyl, C₁₋₆ alkoxyl, C₃₋₆ cycloalkoxyl and 3-6 membered heterocycloalkoxyl, the C₁₋₆ alkyl, C₁₋₆ alkoxyl, C₃₋₆ cycloalkoxyl or 3-6 membered heterocycloalkoxyl is optionally further substituted with one or more groups selected from the group consisting of halogen, deuterium, hydroxy, nitro, cyano, amino and C₁₋₆ alkoxyl;
B is boron atom;
Z is selected from carbon atom and nitrogen atom;
R¹ is each independently selected from the group consisting of hydrogen, halogen, deuterium, hydroxy, oxo, nitro, cyano, amino, C₁₋₆ alkyl, C₃₋₆ cycloalkyl, 3-6 membered heterocyclyl, C₁₋₆ alkoxyl, C₃₋₆ cycloalkoxyl and 3-6 membered heterocycloalkoxyl, the C₁₋₆ alkyl, C₃₋₆ cycloalkyl, 3-6 membered heterocyclyl, C₁₋₆ alkoxyl, C₃₋₆ cycloalkoxyl or 3-6 membered heterocycloalkoxyl is optionally further substituted with one or more of R^{A2};
R^{A2} is selected from the group consisting of halogen, deuterium, hydroxy, nitro, cyano, amino, C₁₋₆ alkyl, C₃₋₆ cycloalkyl, 3-6 membered heterocyclyl, C₁₋₆ alkoxyl, C₃₋₆ cycloalkoxyl and 3-6 membered heterocycloalkoxyl, the C₁₋₆ alkyl, C₁₋₆ alkoxyl, C₃₋₆ cycloalkoxyl or 3-6 membered heterocycloalkoxyl is optionally further substituted with one or more groups selected from the group consisting of halogen, deuterium, hydroxy, nitro, cyano, amino and C₁₋₆ alkoxyl;
R² is selected from the group consisting of hydrogen, alkyl, cycloalkyl, heterocyclyl, aryl and heteroaryl, the alkyl, cycloalkyl, heterocyclyl, aryl or heteroaryl is optionally further substituted with one or more of R^{A3};
R^{A3} is selected from the group consisting of halogen, deuterium, hydroxy, oxo, nitro, cyano and amino;
R³, R⁴ and R⁵ are each independently selected from the group consisting of hydrogen, deuterium, halogen, amino, hydroxy, C₁₋₆ alkyl, C₁₋₆ alkoxyl, C₃₋₆ cycloalkoxyl, C₃₋₆ cycloalkyl, 3-6 membered heterocyclyl and 3-6 membered heterocycloalkoxyl, the alkyl, alkoxyl, cycloalkoxyl, cycloalkyl, heterocyclyl or heterocycloalkoxyl is optionally further substituted with one or more of R^{A4};
R^{A4} is selected from the group consisting of halogen, deuterium, hydroxy, oxo, nitro, cyano and amino;
R⁶ and R⁷, together with the carbon atoms to which they are attached, form a 3-10 membered carbocyclic ring or 3-10 membered heterocyclic ring, the carbocyclic ring or heterocyclic ring is optionally further substituted with one or more of R^{A5};
R^{A5} is selected from the group consisting of halogen, deuterium, hydroxy, oxo, nitro, cyano, C₁₋₆ alkyl, C₁₋₆ alkoxyl, C₃₋₆ cycloalkoxyl, 3-6 membered heterocycloalkoxyl, phenyl and 5-6 membered heteroaryl, the C₁₋₆ alkyl, C₁₋₆ alkoxyl, C₃₋₆ cycloalkoxyl, 3-6 membered heterocycloalkoxyl, C₃₋₈ cycloalkenyloxyl, phenyl or 5-6 membered heteroaryl is optionally further substituted with one or more groups selected from the group consisting of halogen, deuterium, hydroxy, oxo, nitro and cyano;
m is an integer between 0 and 5;
n is an integer between 1 and 3, for example 1 or 2;
and 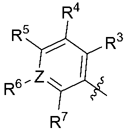 and 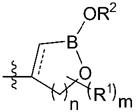 are in the meta-position on ring A;
" / " is a single bond or absent.

In some embodiments, in the compound of formula I or a pharmaceutically acceptable salt thereof, R³ and R⁴ are each independently selected from the group consisting of hydrogen, deuterium, halogen, amino, hydroxy, C₁₋₆ alkyl and C₁₋₆ alkoxyl, the alkyl or alkoxyl is optionally further substituted with one or more of R^{A4}, R^{A4} is as defined above.

In some embodiments, in the compound of formula I or a pharmaceutically acceptable salt thereof, R³ and R⁴ are each independently selected from the group consisting of hydrogen, deuterium, halogen, amino, hydroxy, C₃₋₆ cycloalkoxyl, C₃₋₆ cycloalkyl, 3-6 membered heterocyclyl and 3-6 membered heterocycloalkoxyl, the cycloalkoxyl, cycloalkyl, heterocyclyl or heterocycloalkoxyl is optionally further substituted with one or more of R^{A4}, R^{A4} is as defined above.

In some other embodiments, in the compound of formula I or a pharmaceutically acceptable salt thereof, R⁵ is selected from the group consisting of hydrogen, deuterium, halogen, amino, hydroxy, C₁₋₆ alkyl and C₁₋₆ alkoxyl, the alkyl or alkoxyl is optionally further substituted with one or more of R^{A4}.

In some other embodiments, in the compound of formula I or a pharmaceutically acceptable salt thereof, R⁵ is selected from the group consisting of hydrogen, deuterium, halogen, amino, hydroxy, C₃₋₆ cycloalkoxyl, C₃₋₆ cycloalkyl, 3-6 membered heterocyclyl and 3-6 membered heterocycloalkoxyl, the cycloalkoxyl, cycloalkyl, heterocyclyl or heterocycloalkoxyl is optionally further substituted with one or more of R^{A4}.

Furthermore, in the compound of formula I or a pharmaceutically acceptable salt thereof provided in some embodiments, R³ and R⁴ are each independently selected from hydrogen; R⁵ is selected from the groups consisting of C₁₋₆ alkyl and C₁₋₆ alkoxyl, the alkyl or alkoxyl is optionally substituted with 1 to 3 R^{A4}.

In some embodiments, in the compound of formula I or a pharmaceutically acceptable salt thereof, R^{A4} is selected from halogen, for example fluorine.

In some embodiments, in the compound of formula I or a pharmaceutically acceptable salt thereof, R^{A4} is selected from the group consisting of hydroxy, nitro, cyano and amino.

In another aspect, the compound of formula I provided in some embodiments is 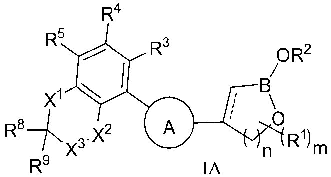
wherein, X¹ is selected from the group consisting of -O-, -N(R^{16a})- and -CR^{16a}R^{16b}-;
X² is selected from the group consisting of -O- and -CR^{17a}R^{17b}-;
X³ is selected from the group consisting of a bond, -CR^{18a}R^{18b}- and - CR^{18a}R^{18b}CR^{18c}R^{18d}-;
R^{16a} and R^{16b} are each independently selected from the group consisting of hydrogen, deuterium, halogen, amino, hydroxy, C₁₋₆ alkyl and C₁₋₆ alkoxyl, the alkyl or alkoxyl is optionally substituted with halogen, nitro, cyano or C₁₋₆ alkoxyl;
R^{17a} and R^{17b} are each independently selected from the group consisting of hydrogen, deuterium, halogen, amino, hydroxy, C₁₋₆ alkyl and C₁₋₆ alkoxyl, the alkyl or alkoxyl is optionally substituted with halogen, nitro, cyano or C₁₋₆ alkoxyl;
R^{18a}, R^{18b}, R^{18c} and R^{18d} are each independently selected from the group consisting of hydrogen, deuterium, halogen, amino, hydroxy, C₁₋₆ alkyl, and C₁₋₆ alkoxyl, the alkyl or alkoxyl is optionally substituted with halogen, nitro, cyano or C₁₋₆ alkoxyl;
R⁸ and R⁹ are each independently selected from the group consisting of hydrogen, deuterium, halogen, amino, hydroxy, C₁₋₆ alkyl, C₁₋₆ alkoxyl, C₃₋₆ cycloalkoxyl, C₃₋₆ cycloalkyl, 3-6 membered heterocyclyl and 3-6 membered heterocycloalkoxyl, the alkyl, alkoxyl, cycloalkoxyl, cycloalkyl or heterocyclyl is optionally further substituted with one or more of R^{A6};
or R⁸ and R⁹, together with the carbon atoms to which they are attached, form a 3-6 membered carbocyclic ring or 3-6 membered heterocyclic ring, the carbocyclic ring or heterocyclic ring is optionally further substituted with one or more of R^{A6};
or R⁸ and R⁹ together form oxo (=O);
R^{A6} is selected from the group consisting of halogen, deuterium, hydroxy, oxo, nitro, cyano, amino, C₁₋₆ alkyl and C₁₋₆ alkoxyl;
ring A, R¹~R⁵, B, m, n and "/" are as defined in the compound of formula I.

In some embodiments, in the compound of formula IA or a pharmaceutically acceptable salt thereof, X¹ is selected from -O-.

In some embodiments, in the compound of formula IA or a pharmaceutically acceptable salt thereof, X¹ is selected from -O-; X² is selected from -O- and -CR^{17a}R^{17b}-; R^{17a} and R^{17b} are each independently selected from the group consisting of hydrogen, deuterium, halogen, amino, hydroxy, C₁₋₆ alkyl and C₁₋₆ alkoxyl.

In some embodiments, in the compound of formula IA or a pharmaceutically acceptable salt thereof, X³ is selected from a bond and -CR^{18a}R^{18b}-; R^{18a} and R^{18b} are each independently selected from the group consisting of hydrogen, deuterium, halogen, amino, hydroxy, C₁₋₆ alkyl and C₁₋₆ alkoxyl.

In some embodiments, in the compound of formula IA or a pharmaceutically acceptable salt thereof, X¹ is selected from -O-; X² is selected from -O- and -CR^{17a}R^{17b}-; R^{17a} and R^{17b} are each independently selected from the group consisting of hydrogen, deuterium, halogen, amino, hydroxy, C₁₋₆ alkyl and C₁₋₆ alkoxyl; X³ is selected from a bond.

In some embodiments, in the compound of formula IA or a pharmaceutically acceptable salt thereof, X¹ is selected from -O-; X² is selected from -O- and -CR^{17a}R^{17b}-; R^{17a} and R^{17b} are each independently selected from the group consisting of hydrogen, deuterium, halogen, amino, hydroxy, C₁₋₆ alkyl and C₁₋₆ alkoxyl; X³ is selected from - CR^{18a}R^{18b}-; R^{18a} and R^{18b} are each independently selected from the group consisting of hydrogen, deuterium, halogen, amino, hydroxy, C₁₋₆ alkyl and C₁₋₆ alkoxyl.

In another aspect, the compound of formula IA provided in some embodiments is

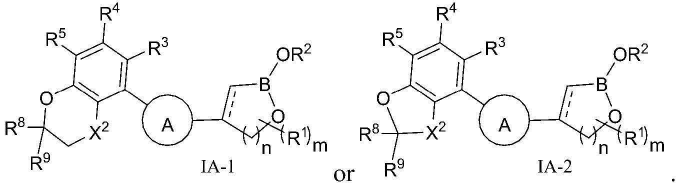

In some embodiments, in the compound of formula I or formula IA or a pharmaceutically acceptable salt thereof, R⁸ and R⁹ are each independently selected from the group consisting of hydrogen, deuterium, halogen, amino, hydroxy, C₁₋₆ alkyl and C₁₋₆ alkoxyl, the alkyl or alkoxyl is optionally further substituted with one or more of R^{A6}; R^{A6} is selected from the group consisting of halogen, deuterium, hydroxy, oxo, nitro, cyano, amino, C₁₋₆ alkyl and C₁₋₆ alkoxyl.

In some embodiments, in the compound of formula IA or a pharmaceutically acceptable salt thereof, the 3-6 membered carbocyclic ring or 4-6 membered heterocyclic ring formed by R⁸ and R⁹ together with the carbon atoms to which they are attached, is selected from the group consisting of 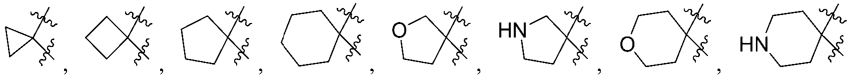 and 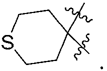 Furthermore, the carbocyclic ring or heterocyclic ring is optionally substituted with 1 to 3 R^{A6}; R^{A6} is selected from the group consisting of halogen, deuterium, hydroxy, oxo, nitro, cyano, amino, C₁₋₆ alkyl and C₁₋₆ alkoxyl.

In some embodiments, in the compound of formula IA or a pharmaceutically acceptable salt thereof, R^{A6} is selected from deuterium and oxo. In some embodiments, in the compound of formula IA or a pharmaceutically acceptable salt thereof, R^{A6} is selected from the group consisting of halogen, C₁₋₆ alkyl and C₁₋₆ alkoxyl. In some embodiments, in the compound of formula IA or a pharmaceutically acceptable salt thereof, R^{A6} is selected from the group consisting of fluorine, chlorine, methyl, ethyl, methoxyl and ethoxyl.

In another aspect, the compound of formula I in some embodiments is 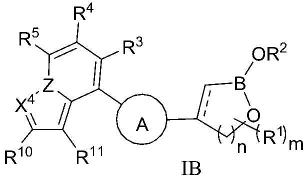
wherein, X⁴ is selected from nitrogen atom and carbon atom;
R¹⁰ and R¹¹ are each independently selected from the group consisting of hydrogen, deuterium, halogen, amino, hydroxy, C₁₋₆ alkyl, C₁₋₆ alkoxyl, C₃₋₆ cycloalkoxyl, C₃₋₆ cycloalkyl, 3-6 membered heterocyclyl and 3-6 membered heterocycloalkoxyl, the alkyl, alkoxyl, cycloalkoxyl, cycloalkyl or heterocyclyl is optionally further substituted with one or more of R^{A7};
R^{A7} is selected from the group consisting of halogen, deuterium, hydroxy, oxo, nitro, cyano, amino, C₁₋₆ alkyl and C₁₋₆ alkoxyl;
ring A, Z, R¹~R⁵, B, m, n and " " are as defined in the compound of formula I.

In some embodiments, in the compound of formula IB or a pharmaceutically acceptable salt thereof, R¹⁰ and R¹¹ are each independently selected from the group consisting of hydrogen, deuterium, halogen, amino, hydroxy, C₁₋₆ alkyl and C₁₋₆ alkoxyl, the alkyl or alkoxyl is optionally further substituted with 1 to 3 R^{A7}. In some embodiments, in the compound of formula IB or a pharmaceutically acceptable salt thereof, R^{A7} is selected from the group consisting of halogen, C₁₋₆ alkyl and C₁₋₆ alkoxyl. In some embodiments, in the compound of formula IB or a pharmaceutically acceptable salt thereof, R^{A7} is selected from the group consisting of fluorine, chlorine, methyl, ethyl, methoxyl and ethoxyl.

In another aspect, in some embodiments, in the compound of formula IB or a pharmaceutically acceptable salt thereof, X⁴ is selected from nitrogen atom; Z is selected from nitrogen atom.

In some embodiments, the compound of formula I is 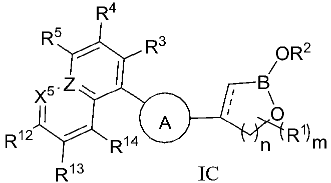
wherein, X⁵ is selected from nitrogen atom and carbon atom;
R¹², R¹³ and R¹⁴ are each independently selected from the group consisting of hydrogen, deuterium, halogen, amino, hydroxy, C₁₋₆ alkyl, C₁₋₆ alkoxyl, C₃₋₆ cycloalkoxyl, C₃₋₆ cycloalkyl, 3-6 membered heterocyclyl and 3-6 membered heterocycloalkoxyl, the alkyl, alkoxyl, cycloalkoxyl, cycloalkyl or heterocyclyl is optionally further substituted with one or more of R^{A8};
R^{A8} is selected from the group consisting of halogen, deuterium, hydroxy, oxo, nitro, cyano, amino, C₁₋₆ alkyl and C₁₋₆ alkoxyl;
ring A, Z, R¹~R⁵, B, m, n and "/" are as defined in the compound of formula I.

In some embodiments, in the compound of formula IC or a pharmaceutically acceptable salt thereof, R¹², R¹³ and R¹⁴ are each independently selected from the group consisting of hydrogen, deuterium, halogen, amino, hydroxy, C₁₋₆ alkyl and C₁₋₆ alkoxyl, the alkyl or alkoxyl is optionally further substituted with 1 to 3 R^{A8}. In some embodiments, in the compound of formula IC or a pharmaceutically acceptable salt thereof, R^{A8} is selected from the group consisting of halogen, C₁₋₆ alkyl and C₁₋₆ alkoxyl. In some embodiments, in the compound of formula IC or a pharmaceutically acceptable salt thereof, R^{A8} is selected from the group consisting of fluorine, chlorine, methyl, ethyl, methoxyl and ethoxyl.

In another aspect, in some embodiments, in the compound of formula IC or a pharmaceutically acceptable salt thereof, X⁵ is selected from nitrogen atom; Z is selected from carbon atom.

In another aspect, in some embodiments, in the compound of formula I or a pharmaceutically acceptable salt thereof, ring A is selected from the group consisting of 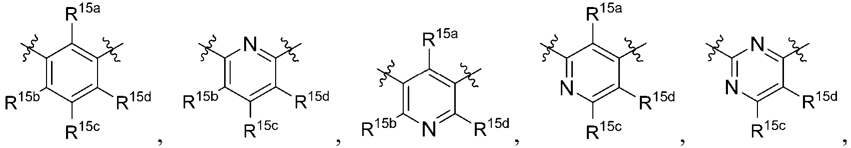 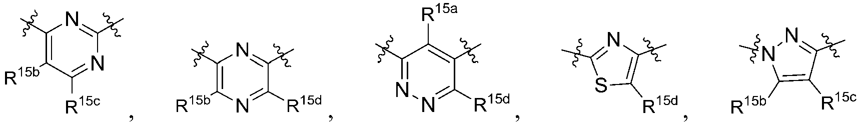 and 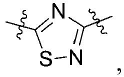 wherein R^{15a}, R^{15b}, R^{15c} and R^{15d} are each independently selected from the group consisting of hydrogen, deuterium, halogen, amino, hydroxy, C₁₋₆ alkyl and C₁₋₆ alkoxyl, the alkyl or alkoxyl is optionally further substituted with one or more of halogen, deuterium, hydroxy, nitro, cyano or amino.

In some embodiments, in the compound of formula I or a pharmaceutically acceptable salt thereof, R^{15a}, R^{15b}, R^{15c} and R^{15d} are each independently selected from the group consisting of hydrogen, deuterium, halogen and C₁₋₆ alkyl. In some embodiments, in the compound of formula I or a pharmaceutically acceptable salt thereof, R^{15a}, R^{15b}, R^{15c} and R^{15d} are each independently selected from the group consisting of fluorine, chlorine, methyl and ethyl.

In the compound of formula I or a pharmaceutically acceptable salt thereof provided in some other embodiments, ring A is selected from the group consisting of: 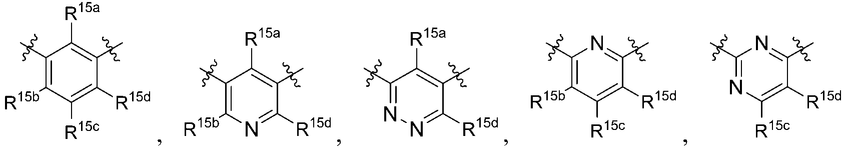 and

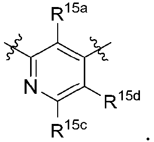

In some embodiments, in the compound of formula I or a pharmaceutically acceptable salt thereof, n is selected from 1 and 2.

In some embodiments, the compound of formula I or formula IA is selected from the group consisting of

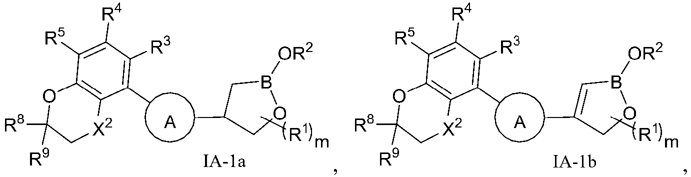

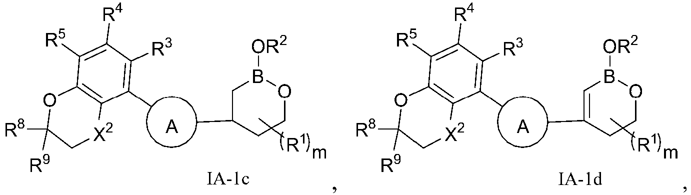

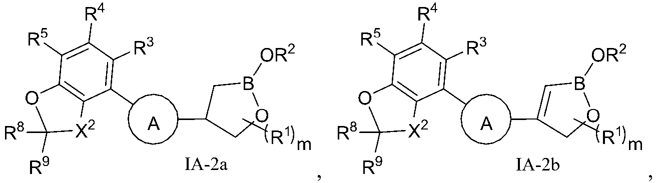

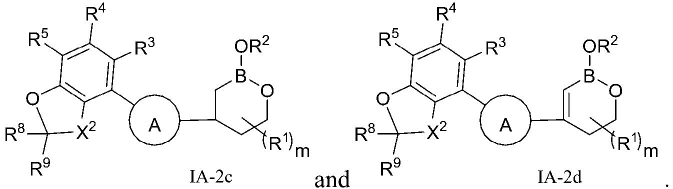

In some embodiments, the compound of formula I or formula IA is selected from the group consisting of

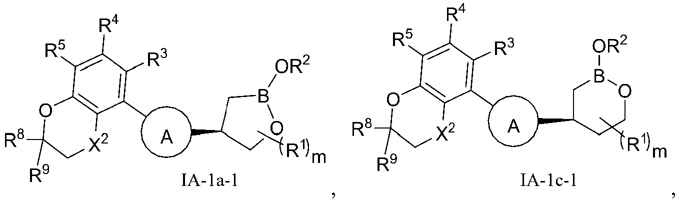

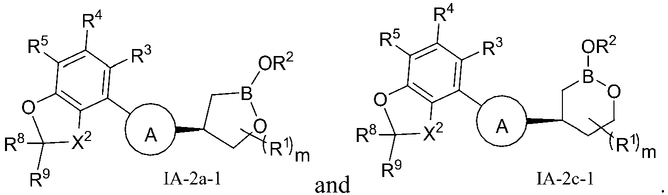

In some embodiments, the compound of formula I or formula IB is selected from

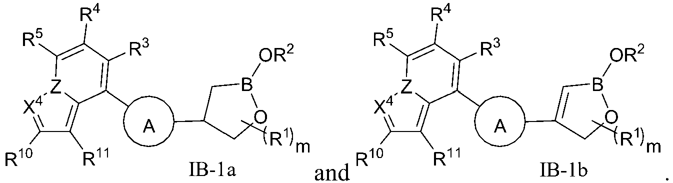

In some embodiments, the compound of formula I or formula IC is selected from

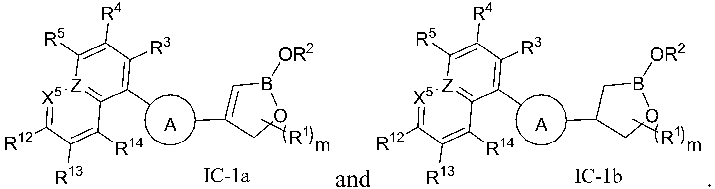

In some embodiments, the compound of formula I is selected from

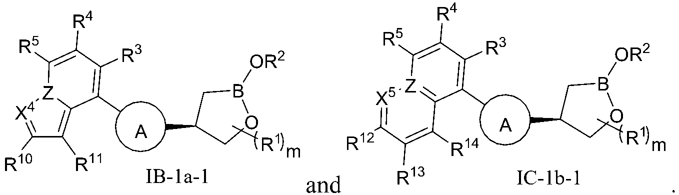

In another aspect, in the compound of formula I or formula IA or formula IB or formula IC or a pharmaceutically acceptable salt thereof provided in some embodiments, R² is selected from the group consisting of hydrogen and C₁₋₆ alkyl. In some embodiments, in the compound of formula I or formula IA or formula IB or formula IC or a pharmaceutically acceptable salt thereof, R² is selected from the group consisting of hydrogen, methyl and ethyl. In some embodiments, in the compound of formula I or formula IA or formula IB or formula IC or a pharmaceutically acceptable salt thereof, R² is selected from hydrogen.

In another aspect, in the compound of formula I or formula IA or formula IB or formula IC or a pharmaceutically acceptable salt thereof provided in some embodiments, R¹ is each independently selected from the group consisting of hydrogen, deuterium, halogen, amino, hydroxy, C₁₋₆ alkyl and C₁₋₆ alkoxyl, the alkyl or alkoxyl is optionally further substituted with one or more groups consisting of halogen, deuterium, hydroxy, nitro, cyano and amino. In some embodiments, in the compound of formula I or formula IA or formula IB or formula IC or a pharmaceutically acceptable salt thereof, R¹ is each independently selected from the group consisting of hydrogen, fluorine, chlorine, methyl, ethyl, methoxyl and ethoxyl.

The compound of formula I provided in some other embodiments is

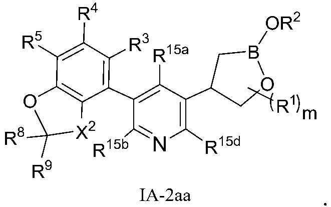

In some embodiments, the compound of formula I is

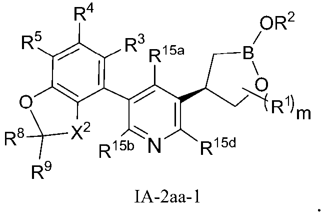

The compound of formula I provided in some other embodiments is selected from the group consisting of

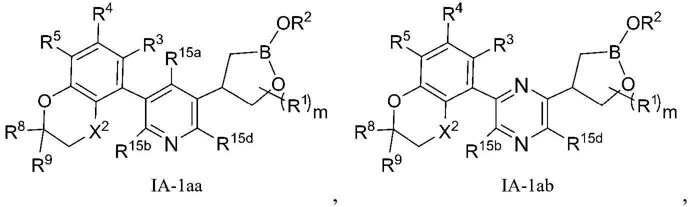

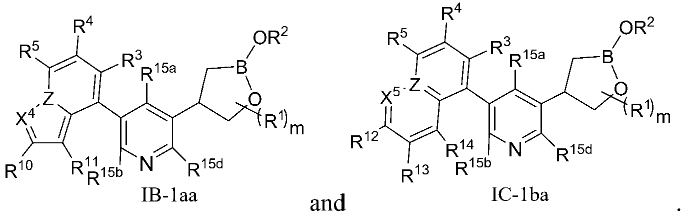

In some embodiments, the compound of formula I is selected from the group consisting of

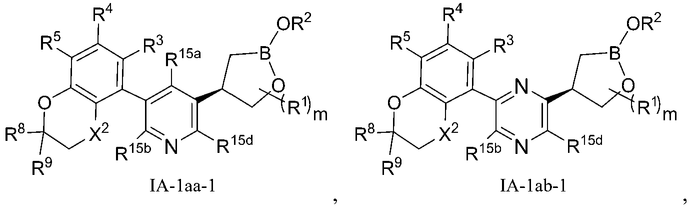

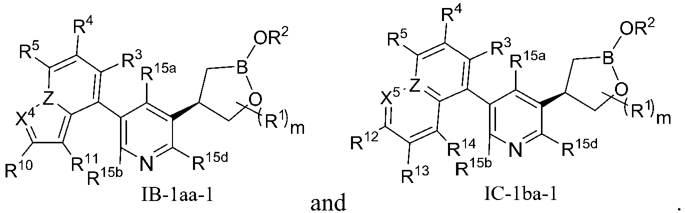

Typical compounds of formula I or a pharmaceutically acceptable salt thereof include, but are not limited to:

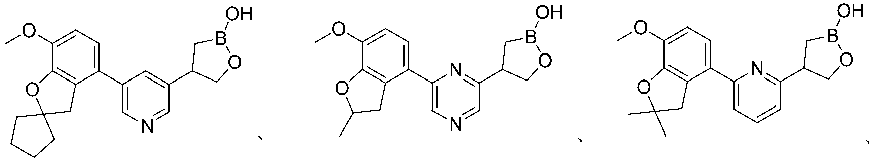

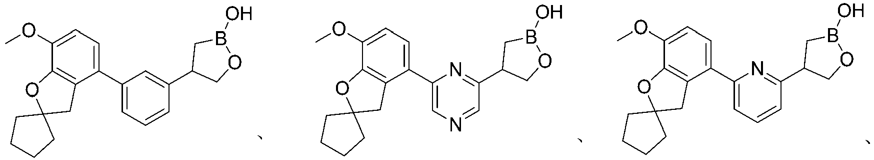

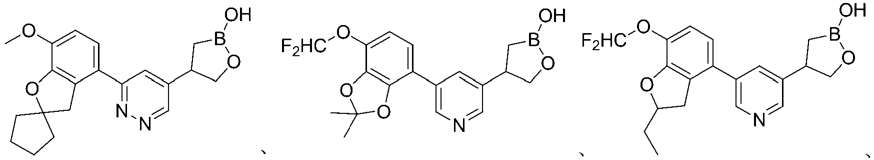

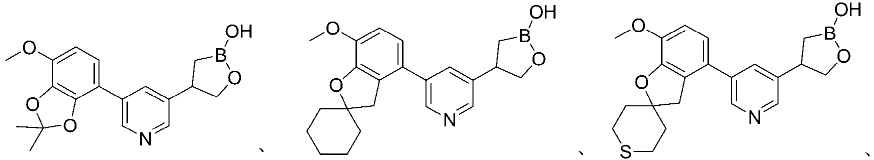

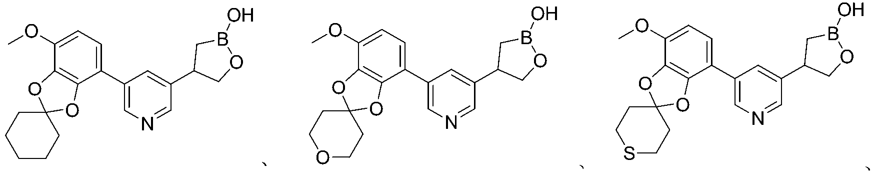

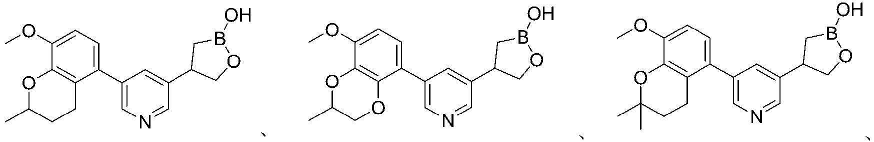

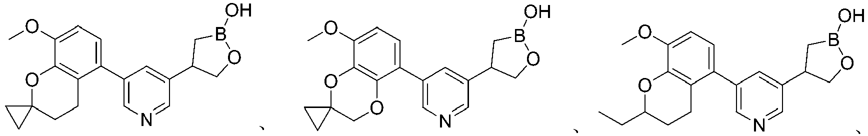

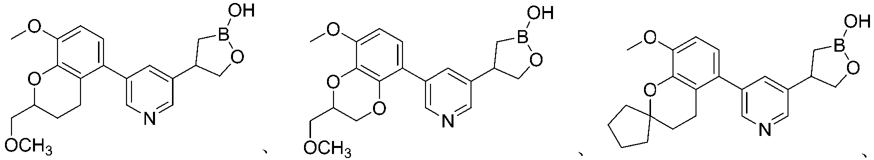

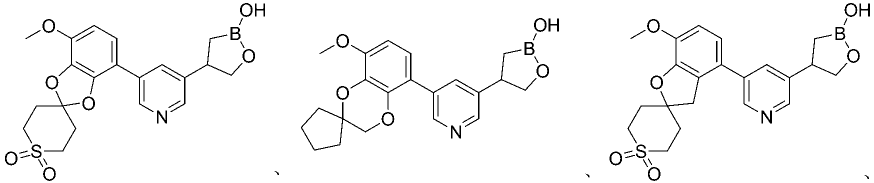

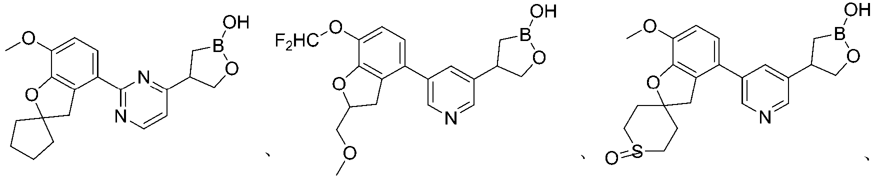

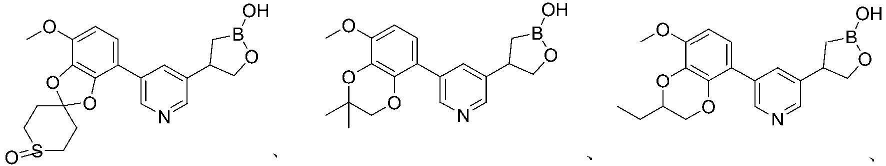

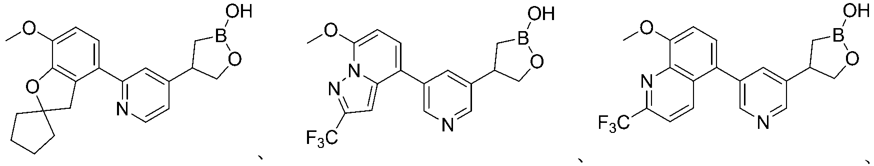

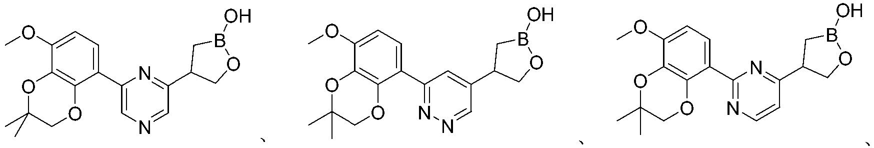

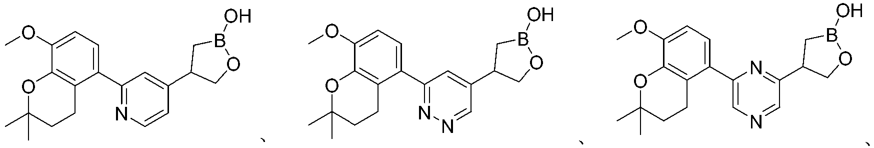

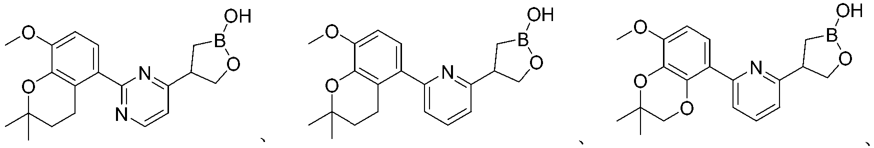

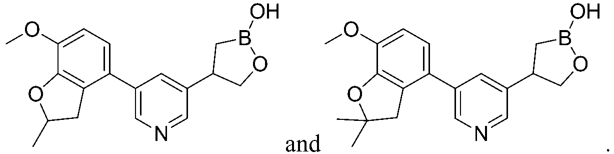

Typical compounds of formula I or a pharmaceutically acceptable salt thereof include, but are not limited to:

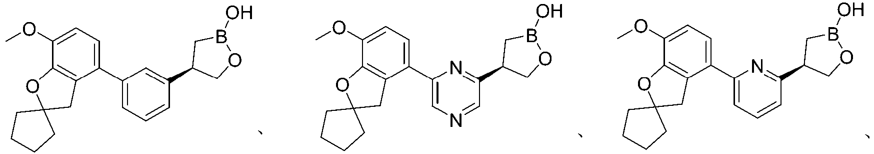

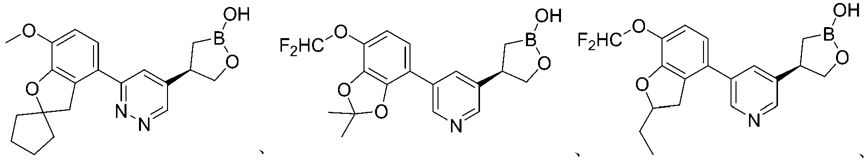

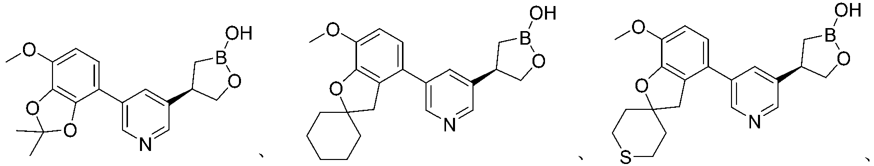

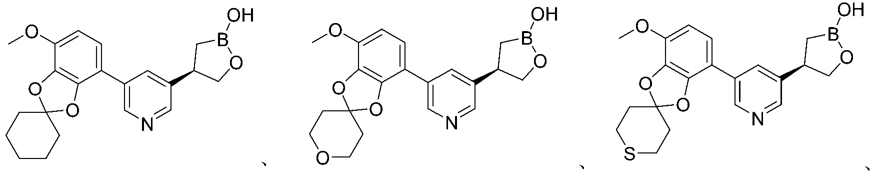

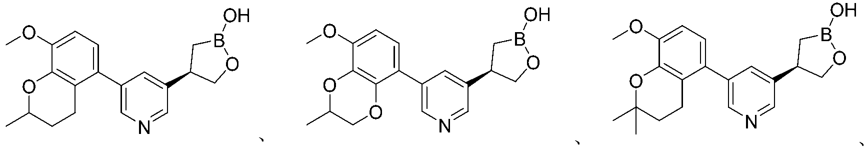

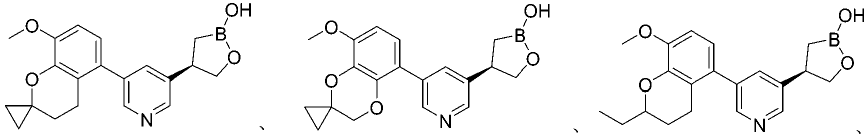

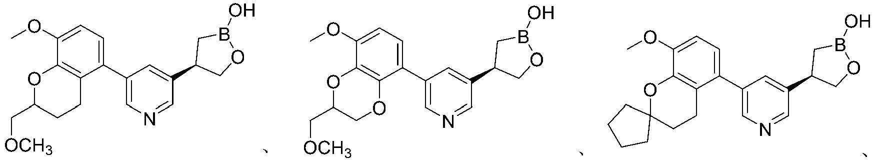

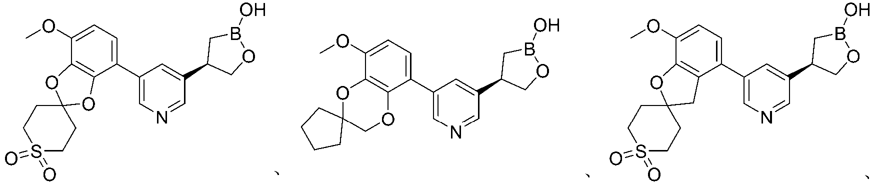

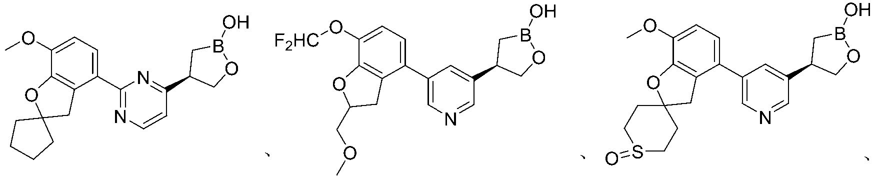

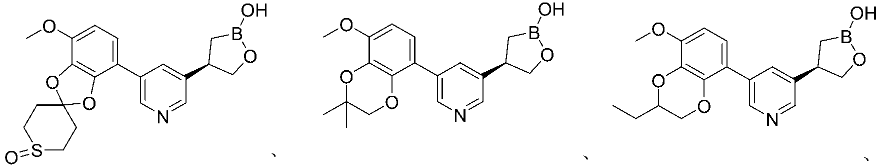

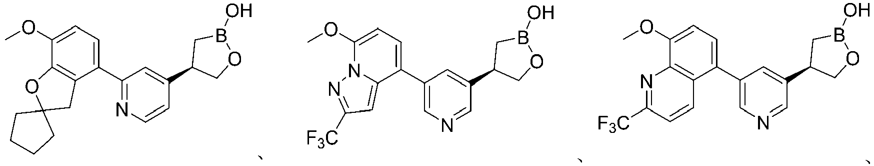

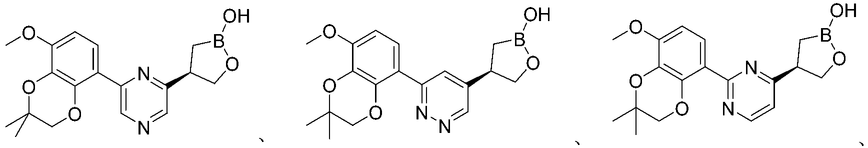

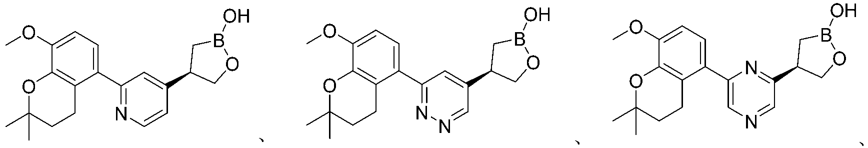

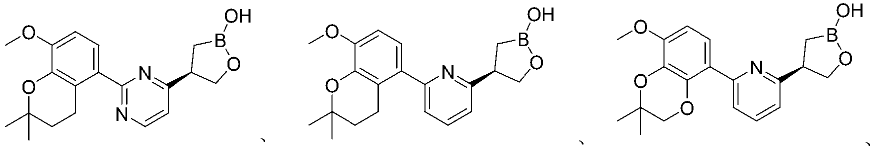

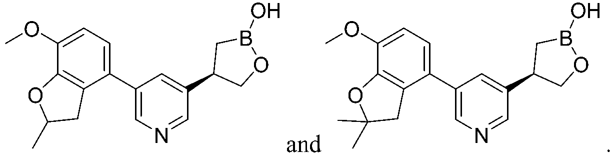

In another aspect, the present disclosure also provides a compound of formula (1) or a pharmaceutically acceptable salt thereof, 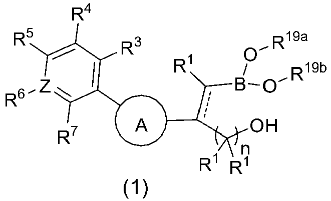 wherein, R^{19a} and R^{19b} are each independently selected from the group consisting of hydrogen and C₁₋₆ alkyl, the alkyl is optionally further substituted with one or more groups selected from the group consisting of halogen, deuterium, hydroxy, oxo, nitro, cyano, amino and C₁₋₆ alkoxyl, or R^{19a} and R^{19b}, together with the atoms to which they are attached, form a 5 membered or 6 membered heterocyclic ring, the heterocyclic ring is optionally further substituted with one or more of R^{A9}, R^{A9} is selected from the group consisting of halogen, deuterium, hydroxy, oxo, nitro, cyano, amino, C₁₋₆ alkyl and C₁₋₆ alkoxyl; ring A, R¹~R⁷, B, n, Z are as defined in the compound of formula I.

In some embodiments, the compound of formula (1) is 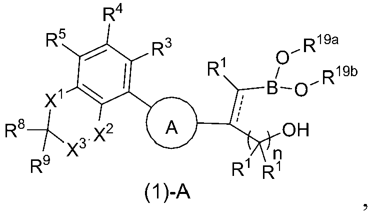 wherein ring A, R¹, R³~R⁵, B, n are as defined in the compound of formula I, R⁸~R⁹, X¹, X², X³ are as defined in the compound of formula IA.

In some embodiments, the compound of formula (1) or a pharmaceutically acceptable salt thereof is 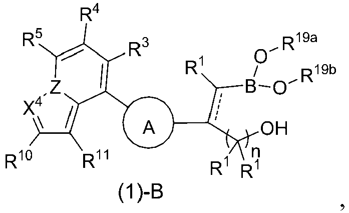 wherein ring A, R¹, R³~R⁵, B, n, Z are as defined in the compound of formula I, R¹⁰~R¹¹, X⁴ are as defined in the compound of formula IB.

In some embodiments, the compound of formula (1) is 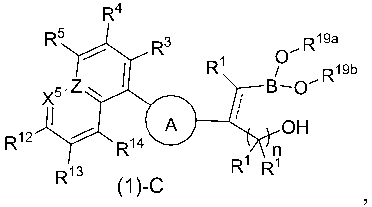 wherein ring A, R¹, R³~R⁵, B, n, Z are as defined in the compound of formula I, R¹²~R¹⁴, X⁵ are as defined in the compound of formula IB.

In another aspect, in some embodiments, the compound of formula (1) is 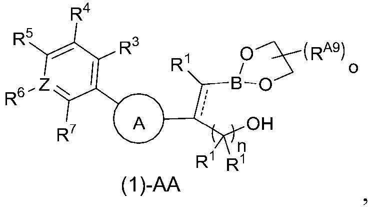 wherein R^{A9} is selected from the group consisting of halogen, deuterium, hydroxy, oxo, nitro, cyano, amino, C₁₋₆ alkyl and C₁₋₆ alkoxyl, o is an integer between 0 and 4, ring A, R¹~R⁷, B, n, Z are as defined in the compound of formula I.

In another aspect, in some embodiments, the compound of formula (1) is 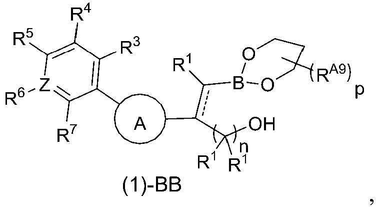 wherein R^{A9} is selected from the group consisting of halogen, deuterium, hydroxy, oxo, nitro, cyano, amino, C₁₋₆ alkyl and C₁₋₆ alkoxyl, p is an integer between 0 and 6, ring A, R¹~R⁷, B, n, Z are as defined in the compound of formula I.

The present disclosure also provides a method for preparing the compound of formula I or a pharmaceutically acceptable salt thereof, comprising the step of converting the compound of formula (1) to the compound of formula I or a pharmaceutically acceptable salt thereof, 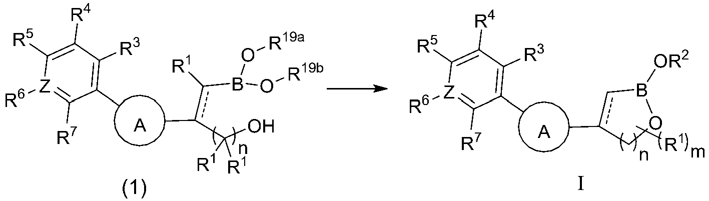 wherein, R^{19a} and R^{19b} are each independently selected from the group consisting of hydrogen and C₁₋₆ alkyl, the alkyl is optionally further substituted with one or more groups selected from the group consisting of halogen, deuterium, hydroxy, oxo, nitro, cyano, amino and C₁₋₆ alkoxyl, or R^{19a} and R^{19b}, together with the atoms to which they are attached, form a 5 membered or 6 membered heterocyclic ring, the heterocyclic ring is optionally further substituted with one or more of R^{A9}, R^{A9} is selected from the group consisting of halogen, deuterium, hydroxy, oxo, nitro, cyano, amino, C₁₋₆ alkyl and C₁₋₆ alkoxyl.

In another aspect, the present disclosure also provides a compound of formula (2) or a pharmaceutically acceptable salt thereof, 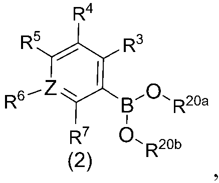 wherein R^{20a} and R^{20b} are each independently selected from the group consisting of hydrogen and C₁₋₆ alkyl, the alkyl is optionally further substituted with one or more groups selected from the group consisting of halogen, deuterium, hydroxy, oxo, nitro, cyano, amino and C₁₋₆ alkoxyl, or R^{20a} and R^{20b}, together with the atoms to which they are attached, form a 5 membered or 6 membered heterocyclic ring, the heterocyclic ring is optionally further substituted with one or more of R^{A10}, R^{A10} is selected from the group consisting of halogen, deuterium, hydroxy, oxo, nitro, cyano, amino, C₁₋₆ alkyl and C₁₋₆ alkoxyl.

The compound of formula (2) provided in some embodiments is

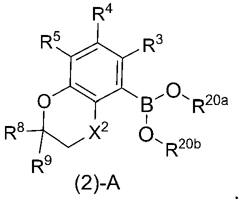

The compound of formula (2) provided in some embodiments is

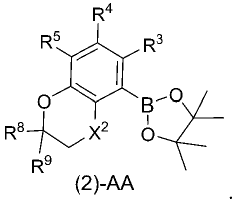

In some embodiments, the compound of formula (2) or a pharmaceutically acceptable salt thereof is selected from the group consisting of: 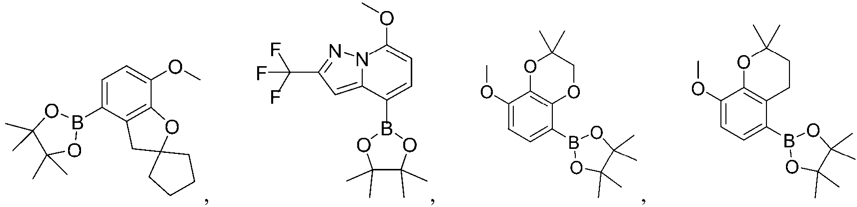 and

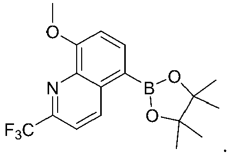

The present disclosure also provides a pharmaceutical composition comprising a therapeutically amount of at least one of the aforementioned compound of formula I or a pharmaceutically acceptable salt thereof, as well as a pharmaceutically acceptable excipient.

In some embodiments, the unit dose of the pharmaceutical composition is 0.001 mg-1000 mg.

In certain embodiment, based on the total weight of the composition, the pharmaceutical composition contains 0.01%-99.99% of the aforementioned compound or a pharmaceutically acceptable salt thereof. In certain embodiment, the pharmaceutical composition contains 0.1%-99.9% of the aforementioned compound or a pharmaceutically acceptable salt thereof. In certain embodiment, the pharmaceutical composition contains 0.5%-99.5% of the aforementioned compound or a pharmaceutically acceptable salt thereof. In certain embodiment, the pharmaceutical composition contains 1%-99% of the aforementioned compound or a pharmaceutically acceptable salt thereof. In certain embodiment, the pharmaceutical composition contains 2%-98% of the aforementioned compound or a pharmaceutically acceptable salt thereof.

In certain embodiment, based on the total weight of the composition, the pharmaceutical composition contains 0.01%-99.99% of the pharmaceutically acceptable excipient. In certain embodiment, the pharmaceutical composition contains 0.1%-99.9% of the pharmaceutically acceptable excipient. In certain embodiment, the pharmaceutical composition contains 0.5%-99.5% of the pharmaceutically acceptable excipient. In certain embodiments, the pharmaceutical composition contains 1%-99% of the pharmaceutically acceptable excipient. In certain embodiments, the pharmaceutical composition contains 2%-98% of the pharmaceutically acceptable excipient.

The present disclosure also provides a method of preventing and/or treating a patient with PDE-related disorders, by administering to the patient a therapeutically effective amount of the aforementioned compound of formula I or formula IA or a pharmaceutically acceptable salt thereof, or a compound or a pharmaceutically acceptable salt thereof prepared by the aforementioned method, or the aforementioned pharmaceutical composition.

In some embodiments, the PDE-related disorder is preferably asthma, obstructive pulmonary disease, septicaemia, nephritis, diabetes, allergic rhinitis, allergic conjunctivitis, ulcerative enteritis or rheumatism.

The present disclosure also provides a method of preventing and/or treating a patient with asthma, obstructive pulmonary disease, septicaemia, nephritis, diabetes, allergic rhinitis, allergic conjunctivitis, ulcerative enteritis or rheumatism, by administering to the patient a therapeutically effective amount of the aforementioned compound of formula I or formula IA or a pharmaceutically acceptable salt thereof, or a compound or a pharmaceutically acceptable salt thereof prepared by the aforementioned method, or the aforementioned pharmaceutical composition.

The present disclosure also provides use of the aforementioned compound of formula I or formula IA or a pharmaceutically acceptable salt thereof, or the aforementioned pharmaceutical composition, in the preparation of a medicament for preventing and/or treating PDE-related disorders. In some embodiments, the PDE-related disorder is preferably asthma, obstructive pulmonary disease, septicaemia, nephritis, diabetes, allergic rhinitis, allergic conjunctivitis, ulcerative enteritis or rheumatism.

The present disclosure also provides use of the aforementioned compound of formula I or formula IA or a pharmaceutically acceptable salt thereof, or the aforementioned pharmaceutical composition, in the preparation of a medicament for preventing and/or treating asthma, obstructive pulmonary disease, septicaemia, nephritis, diabetes, allergic rhinitis, allergic conjunctivitis, ulcerative enteritis or rheumatism.

In another aspect, the pharmaceutically acceptable salt of the compound described in the present disclosure is selected from inorganic salt and organic salt.

The compound of the present disclosure may exist in a specific geometric or stereoisomer form. The present disclosure considers all such compounds, including cis- and trans-isomers, (-)- and (+)-enantiomers, (R)- and (S)-enantiomers, diastereomers, (D)-isomers, (L)-isomers, and racemic mixtures and other mixtures, such as enantiomer- or diastereomer-enriched mixtures, all of which fall within the scope of the present disclosure. Additional asymmetric carbon atoms may exist in substituent groups such as alkyl. All of these isomers and mixtures thereof are included within the scope of the present disclosure. The compound containing asymmetric carbon atoms of the present disclosure can be isolated in an optical pure form or racemic form. The optical pure form can be separated from racemic mixtures or synthesized by using chiral raw materials or chiral reagents.

Optically active (R)- and (S)- isomers as well as D and L isomers can be prepared by chiral synthesis or chiral reagents or other conventional techniques. If one type of enantiomer of a certain compound of the present disclosure is desired, it can be prepared by asymmetric synthesis or derivation with chiral additives, wherein the resulting enantiomer mixture is separated, and the auxiliary group is cleaved to provide the pure desired enantiomer. Alternatively, when the molecule contains a basic functional group (such as amino) or an acidic functional group (such as carboxyl), a diastereomer salt is formed with an appropriate optically active acid or base. Then the diastereomer is resolved by conventional methods well known in the art, and then the pure enantiomer is recovered. Furthermore, the separation of enantiomers and diastereomers is typically carried out by using chromatography, which employs a chiral stationary phase and optionally combined with chemical derivatization (e.g., generating carbamates from amines).

In the chemical structure of the compound according to the present disclosure, the bond "/" means unspecified configuration, that is, if chiral isomers exist in the chemical structure, the bond "/" can be " 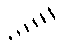 " or " 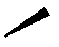 ", or includes the two configurations " 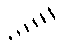 " and " 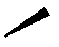 " at the same time. The bond " 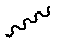 " means unspecified configuration, including cis- (E) and trans- (Z) configurations.

The compound and intermediate of the present disclosure may also exist in different tautomer forms, and all such forms are included within the scope of the present disclosure. The term "tautomer" or "tautomer form" refers to structural isomers of different energies that can be interconverted via a low-energy barrier. For example, proton tautomers (also known as prototropic tautomers) include tautomerism via proton transfer, such as ketone-enol and imine-enamine, lactam-lactim tautomerism. An example of lactam-lactim equilibrium is between A and B as shown below.

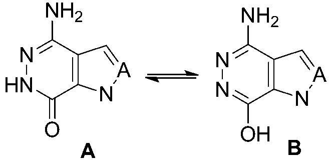

All compounds in the present disclosure can be drawn as type A or type B. All forms of tautomerism are within the scope of the present disclosure. The nomenclature of the compound does not exclude any tautomers.

The present disclosure also includes some isotope-labeled compounds of the present disclosure, which are the same as those described herein, but one or more atoms are replaced with atoms with atomic weight or mass number different from that commonly found in nature. Examples of isotopes that can be incorporated into the compound of the present disclosure include isotopes of hydrogen, carbon, nitrogen, oxygen, phosphorus, sulfur, fluorine, iodine and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively, etc.

Unless otherwise specified, when a position is specifically designated as deuterium (D), this position should be understood as having a deuterium abundance of at least 1,000 folds greater than the natural deuterium abundance (which is 0.015%) (i.e., at least 10% of deuterium incorporation). For example, the deuterium abundance of the compound, which is greater than the natural deuterium abundance, can be a deuterium abundance of at least 1,000 folds, at least 2,000 folds, at least 3,000 folds, at least 4,000 folds, at least 5,000 folds, at least 6,000 folds greater than the natural deuterium abundance, or a higher deuterium abundance. The present disclosure also comprises various deuterated forms of the compound of formula (I). Each available hydrogen atom linked to a carbon atom can be independently substituted with a deuterium atom. Those skilled in the art can synthesize deuterated forms of the compound of formula (I) with reference to relevant literatures. The deuterated forms of the compound of formula (I) can be prepared using commercially available deuterated starting materials, or they can be synthesized by conventional techniques using deuterated reagents, including but not limited to deuterated borane, a solution of trideuterated borane in tetrahydrofuran, lithium aluminum deuteride, deuterated iodoethane and deuterated iodidomethane and the like.

"Optionally" or "optional" means that the event or circumstance described subsequently can, but need not occur, and such a description includes the situation in which the event or circumstance does or does not occur. For example, "the C₁₋₆ alkyl optionally substituted with halogen or cyano" means that halogen or cyano can, but need not exist, and such a description includes the situation in which the alkyl is substituted with halogen or cyano and the situation in which the alkyl is not substituted with halogen or cyano.

The "pharmaceutical composition" refers to a mixture containing one or more of the compounds described herein, or a physiologically pharmaceutically acceptable salt or a prodrug thereof, and other chemical components, as well as other components, such as a physiologically pharmaceutically acceptable carrier and excipient. The objective of the pharmaceutical composition is to facilitate drug administration to an organism, and to benefit the absorption of the active ingredient so as to exert the biological activity.

The "pharmaceutically acceptable excipient" or "acceptable excipient" includes, but is not limited to, any adjuvant, carrier, glidant, sweetener, diluent, preservative, dye/colorant, flavor enhancer, surfactant, wetting agent, dispersant, suspending agent, stabilizer, isotonic agent, solvent or emulsifier that has been approved by the U.S. Food and Drug Administration as acceptable for use in humans or livestock animals.

The "effective amount" or "therapeutically effective amount" according to the present disclosure includes an amount sufficient to ameliorate or prevent the symptoms or conditions of the medical disease. The effective amount also refers to an amount sufficient to allow or facilitate diagnosis. The effective amount for a particular patient or veterinary subject can vary depending on the following factors: such as the condition to be treated, the general health condition of the patient, the method, route and dose of drug administration, and the severity of side effects. The effective amount can be the maximum dose or dosing regimen that avoids significant side effects or toxic effects.

The "alkyl" refers to a saturated aliphatic hydrocarbon group, which is a straight or branched group comprising 1 to 20 carbon atoms. An alkyl contains 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof and the like. The alkyl can be substituted or unsubstituted. When substituted, the substituent group(s) can be substituted at any available connection point. The substituent group(s) is preferably one or more groups independently selected from the group consisting of halogen, deuterium, hydroxy, nitro, cyano, amino, C₁₋₆ alkyl, C₃₋₆ cycloalkyl, 3-6 membered heterocyclyl, C₁₋₆ alkoxyl, C₃₋₆ cycloalkoxyl and 3-6 membered heterocycloalkoxyl, the C₁₋₆ alkyl, C₁₋₆ alkoxyl, C₃₋₆ cycloalkoxyl or 3-6 membered heterocycloalkoxyl is optionally further substituted with one or more groups selected from the group consisting of halogen, deuterium, hydroxy, nitro, cyano, amino and C₁₋₆ alkoxyl.

The term "cycloalkyl" or "carbocyclic ring" refers to a saturated or partially unsaturated monocyclic or polycyclic hydrocarbon substituent. The cycloalkyl ring comprises 3 to 20 carbon atoms, preferably 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl and the like. Polycyclic cycloalkyl includes a cycloalkyl having a spiro ring, fused ring or bridged ring. The cycloalkyl can be substituted or unsubstituted. When substituted, the substituent group(s) can be substituted at any available connection point. The substituent group(s) is preferably one or more groups independently selected from the group consisting of halogen, deuterium, hydroxy, nitro, cyano, amino, C₁₋₆ alkyl, C₃₋₆ cycloalkyl, 3-6 membered heterocyclyl, C₁₋₆ alkoxyl, C₃₋₆ cycloalkoxyl and 3-6 membered heterocycloalkoxyl, the C₁₋₆ alkyl, C₁₋₆ alkoxyl, C₃₋₆ cycloalkoxyl or 3-6 membered heterocycloalkoxyl is optionally further substituted with one or more groups selected from the group consisting of halogen, deuterium, hydroxy, nitro, cyano, amino and C₁₋₆ alkoxyl. The cycloalkyl ring can be fused to an aryl or heteroaryl ring, wherein the ring attached to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more groups independently selected from the group consisting of halogen, deuterium, hydroxy, nitro, cyano, amino, C₁₋₆ alkyl, C₃₋₆ cycloalkyl, 3-6 membered heterocycloalkyl, C₁₋₆ alkoxyl, C₃₋₆ cycloalkoxyl and 3-6 membered heterocycloalkoxyl, the C₁₋₆ alkyl, C₁₋₆ alkoxyl, C₃₋₆ cycloalkoxyl or 3-6 membered heterocycloalkoxyl is optionally further substituted with one or more groups selected from the group consisting of halogen, deuterium, hydroxy, nitro, cyano, amino and C₁₋₆ alkoxyl.

The term "heterocyclyl" refers to a saturated or partially unsaturated monocyclic or polycyclic hydrocarbon substituent comprising 3 to 6 ring atoms. Non-limiting examples of "heterocyclyl" include 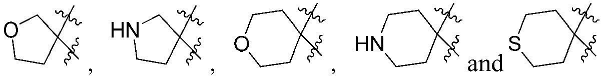 and the like.

The heterocyclyl can be optionally substituted or unsubstituted. When substituted, the substituent group(s) is preferably one or more groups independently selected from the group consisting of halogen, deuterium, hydroxy, oxo, nitro, cyano, C₁₋₆ alkyl, C₁₋₆ alkoxyl, C₃₋₆ cycloalkoxyl, 3-6 membered heterocycloalkoxyl, phenyl and 5-6 membered heteroaryl, the C₁₋₆ alkyl, C₁₋₆ alkoxyl, C₃₋₆ cycloalkoxyl, 3-6 membered heterocycloalkoxyl, C₃₋₈ cycloalkenyloxyl, phenyl or 5-6 membered heteroaryl is optionally further substituted with one or more groups selected from the group consisting of halogen, deuterium, hydroxy, oxo, nitro and cyano.

The term "heteroaryl" refers to a heteroaromatic system having 1 to 4 heteroatoms selected from the group consisting of oxygen, sulfur and nitrogen and 5 to 14 ring atoms. The heteroaryl is preferably 5 membered or 6 membered. For example, its non-limiting examples include 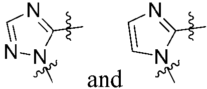 and the like.

The heteroaryl can be optionally substituted or unsubstituted. When substituted, the substituent group(s) is preferably one or more groups independently selected from the group consisting of halogen, deuterium, hydroxy, oxo, nitro, cyano, C₁₋₆ alkyl, C₁₋₆ alkoxyl, C₃₋₆ cycloalkoxyl, 3-6 membered heterocycloalkoxyl, phenyl and 5-6 membered heteroaryl, the C₁₋₆ alkyl, C₁₋₆ alkoxyl, C₃₋₆ cycloalkoxyl, 3-6 membered heterocycloalkoxyl, C₃₋₈ cycloalkenyloxyl, phenyl or 5-6 membered heteroaryl is optionally further substituted with one or more groups selected from the group consisting of halogen, deuterium, hydroxy, oxo, nitro and cyano.

The term "alkoxyl" refers to -O-(alkyl), wherein the alkyl is as defined above. Non-limiting examples of alkoxyl include methoxyl, ethoxyl, propoxyl, butoxyl. The alkoxyl can be optionally substituted or unsubstituted. When substituted, the substituent group(s) is preferably one or more groups independently selected from the group consisting of halogen, deuterium, hydroxy, oxo, nitro, cyano, C₁₋₆ alkyl, C₁₋₆ alkoxyl, C₃₋₆ cycloalkoxyl, 3-6 membered heterocycloalkoxyl, phenyl and 5-6 membered heteroaryl, the C₁₋₆ alkyl, C₁₋₆ alkoxyl, C₃₋₆ cycloalkoxyl, 3-6 membered heterocycloalkoxyl, C₃₋₈ cycloalkenyloxyl, phenyl or 5-6 membered heteroaryl is optionally further substituted with one or more groups selected from the group consisting of halogen, deuterium, hydroxy, oxo, nitro and cyano.

The term "cycloalkoxyl" refers to -O-(cycloalkyl), wherein the cycloalkyl is as defined above, including but not limited to cyclopropyl, cyclobutyl, cyclopentyl, cyclopentenyl and cyclohexyl.

The term "heterocyclic ring" means the atoms that form the ring include other atoms in addition to the carbon atoms, and it includes heterocyclyl and heteroaryl ring.

The term "hydroxyl" refers to an -OH group.

The term "halogen" refers to fluorine, chlorine, bromine or iodine.

The term "cyano" refers to -CN group.

The term "amino" refers to -NH₂ group.

The term "nitro" refers to -NO₂ group.

The term "oxo" refers to the substituent group =O group.

"Substituted" refers to one or more hydrogen atoms in the group, preferably up to 5, and more preferably 1 to 3 hydrogen atoms, each independently substituted by the corresponding number of substituent(s). It goes without saying that the substituents are only in their possible chemical positions. Those skilled in the art are able to determine (by experiment or theory) possible or impossible substitutions without excessive effort.

### DESCRIPTION OF THE DRAWINGS

Figure 1: Clinical score comparison of the compound of each group on a disease model.
Figure 2: Clinical score comparison of the compound of each group on erythema disease model.
Figure 3: Clinical score comparison of the compound of each group on psoriasis disease model.
Figure 4: Comparison of the inhibitory effect of the compound of each group on the increase in skin thickness.
Figure 5: Comparison of the effects of the compound of each group on the ratio of spleen weight to body weight.

### DETAILED DESCRIPTION OF THE INVENTION

The examples are incorporated below for further description of the present disclosure, but these examples do not limit the scope of the present disclosure.

The experimental methods with unspecified conditions in the examples of the present disclosure generally follow conventional conditions or are according to the conditions recommended by the raw material or commodity manufacturer. The reagents without giving particular sources are conventional reagents purchased on the market.

The structures of the compounds are identified by nuclear magnetic resonance (NMR) and/or mass spectrometry (MS). NMR shift is given in 10⁻⁶ (ppm). NMR is determined by a Bruker AVANCE-400 nuclear magnetic spectrometer. The solvents for determination are deuterated dimethyl sulfoxide (DMSO-*d₆*), deuterated chloroform (CDCl₃), deuterated methanol (Methanol-*d₄*), and the internal standard is tetramethylsilane (TMS).

HPLC is determined by an Agilent1100 high pressure liquid chromatograph with a GAS15B DAD UV Detector and Water Vbridge C18 150*4.6 mm 5 µm columns.

MS is determined by an Agilent6120 Triple Quadrupole Mass Spectrometer with G1315D DAD Detector and Waters Xbridge C18 4.6*50 mm, 5 µm columns, scanning in positive/negative ion mode with a mass scanning range of 80~1200.

Preparative HPLC conditions: Waters; Column: Sunfire (Prep C18 OBD 19*250 mm 10 µm);

Chiral column resolution conditions: Column: Chiralpak IG 5 µm 30*250 mm; Mobile Phase: Hex : EtOH = 35 : 65 at 15 mL/min; Temp: 30°C; Wavelength: 254 nm.

Yantai Huanghai HSGF254 silica gel plates are used as the gel plates for thin layer chromatography. The specification is 0.2 mm ± 0.03 mm for thin layer chromatography (TLC), and 0.4 mm - 0.5 mm for separation and purification of products by thin layer chromatography.

Combiflash Rf150 (TELEDYNE ISCO) or Isolara one (Biotage) is used as the rapid column purification system.

In normal-phase column chromatography, Yantai Huanghai silica gel 200~300 mesh or 300~400 mesh silica gel is generally used as the carrier, or Changzhou Santai prefilled ultra-pure normal-phase silica gel columns (40-63 µm, 60 g, 24 g, 40 g, 120 g or other specifications) are used.

The known starting materials of the present disclosure can be synthesized by or according to methods known in the art, or can be purchased from Shanghai Titan, ABCR GmbH & Co. KG, Acros Organics, Aldrich Chemical Company, Accela ChemBio Inc, Bide Pharm and other companies.

Unless specified in the examples, all reactions can be carried out under nitrogen atmosphere.

Nitrogen atmosphere means that the reaction flask is connected to a nitrogen balloon with a volume of about 1 L.

Hydrogen atmosphere means that the reaction flask is connected to a hydrogen balloon with a volume of about 1 L.

Hydrogen is produced by a QPH-1L hydrogen generator from Shanghai Quan Pu Scientific Instruments.

The nitrogen atmosphere or hydrogen atmosphere is generally generated by vacuuming and filling with nitrogen or hydrogen, repeating 3 times.

Unless specified in the examples, a solution refers to an aqueous solution.

Unless specified in the examples, the reaction temperature is room temperature, which is 20°C to 30°C.

The monitoring of the reaction process in the examples adopts thin layer chromatography (TLC). The developing agent used in the reaction, the column chromatography eluent system used for purifying the compound and the developing agent system of thin layer chromatography, and the volume ratio of the solvents are adjusted depending on the polarity of the compound. A small amount of basic or acidic reagents such as triethylamine and acetic acid can also be added for adjustment.

### Example 1

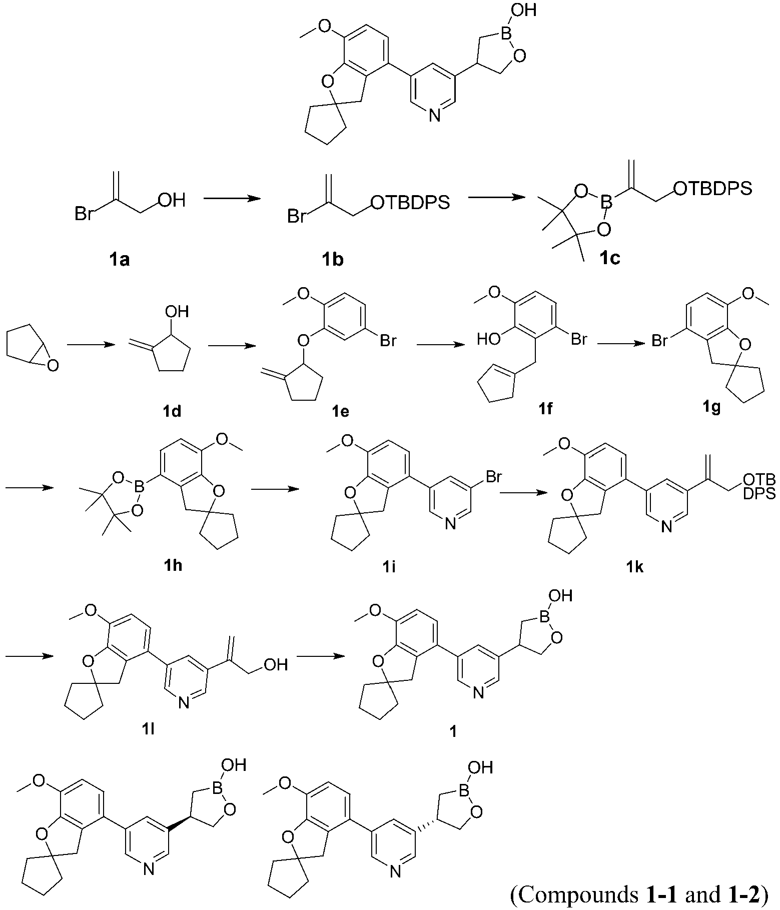

Step 1) Compound **1a** (2.0 g, 14.6 mmol) and triethylamine (1.8 g, 17.8 mmol) were dissolved in N,N-dimethylformamide (30 mL) and the solution was cooled to 0°C. Tert-butyldiphenylchlorosilane (4.0 g, 14.6 mmol) was added dropwise to the reaction system under nitrogen atmosphere. The reaction system was warmed to room temperature and continually stirred until completion of the reaction as detected by TLC. The reaction solution was poured into water and extracted with ethyl acetate (100 mL × 3). The organic phase was washed with water (50 mL × 2), dried over anhydrous sodium sulfate and concentrated. The residues were purified by silica gel column (ethyl acetate/petroleum ether) to obtain Compound **1b** (5.1 g), which was directly used in the next reaction.

Step 2) A mixture of Compound **1b** (5.1 g, 13.6 mmol), bis(pinacolato)diboron (4.2 g, 16.3 mmol), [1,1'-bis(diphenylphosphino)ferrocene]palladium dichloride (512 mg, 0.7 mmol) and potassium acetate (2.0 g, 20.4 mmol) in dioxane (100 mL) was warmed to 80°C and stirred overnight under nitrogen atmosphere. The reaction solution was poured into water and extracted with ethyl acetate (100 mL × 2). The organic phase was washed with water (30 mL × 2), dried over anhydrous sodium sulfate and concentrated. The residues were purified by silica gel column (petroleum ether/ethyl acetate) to obtain Compound **1c** (2.0 g), LCMS: m/z 423.2 (M+H)⁺.

Step 3) Trimethylsulfur iodide (20.93 g, 95.10 mmol) and anhydrous tetrahydrofuran (100 mL) were added to a 250 mL three-neck flask successively, and then stirred until dissolved. The solution was cool to -10°C, and a solution of n-butyl lithium in tetrahydrofuran (35.19 mL, 2.5 M, 87.97 mmol) was slowly added. The mixture was stirred at -10°C for 1 hour. Then a solution of 6-oxabicyclo[3.1.0]hexane (2.00 g, 23.78 mmol) in tetrahydrofuran was slowly added dropwise. After addition, the reaction solution was warmed to room temperature and continually stirred until completion of the reaction as detected by TLC. The reaction solution was slowly poured into water to quench the reaction, and extracted with ethyl acetate (100 mL × 3). The organic phase was dried over anhydrous sodium sulfate and concentrated under reduced pressure to obtain Compound **1d** (400 mg concentrate), which was directly used in the next step.

Step 4) Compound 5 (100.00 mg, 1.02 mmol), 5-bromo-2-methoxyphenol (206.87 mg, 1.02 mmol) and triphenylphosphine (801.12 mg, 3.06 mmol) were added to an anhydrous tetrahydrofuran (10 mL) in a 25 mL single-neck flask successively. The reaction solution was stirred well, purged with nitrogen for 3 times and cooled to 0°C. Diisopropyl azodicarboxylate (618.02 mg, 3.06 mmol) was slowly added dropwise. After the addition, the reaction solution was warmed to room temperature and stirred until completion of the reaction as detected by TLC. 50 mL of water was added to the reaction solution to quench the reaction. The reaction solution was extracted with ethyl acetate (100 mL × 3), dried over anhydrous sodium sulfate and concentrated under reduced pressure. The residues were purified by column chromatography (ethyl acetate/petroleum ether) to obtain Compound **1e** (60 mg).

*¹H* NMR (400 MHz, DMSO-*d₆*) δ 7.17 (*d, J =* 2.3 Hz, 1H), 7.08 (dd, *J =* 8.6, 2.3 Hz, 1H), 6.92 (dd, *J* = 8.6, 4.2 Hz, 1H), 5.05 (d, *J =* 1.5 Hz, 2H), 5.01 (t, *J =* 4.3 Hz, 1H), 3.74 (s, 3H), 2.47-2.36 (m, 1H), 2.35-2.21 (m, 1H), 2.05-1.90 (m, 1H), 1.86-1.59 (m, 3H).

Step 5) Compound **1e** (60.00 mg, 1.02 mmol) was added to a 25 mL single-neck flask, warmed to 180°C and stirred until completion of the reaction as detected by TLC. 10 mL of water was added to the reaction solution to quench the reaction. The reaction solution was extracted with ethyl acetate (20 mL × 3), dried over anhydrous sodium sulfate and concentrated under reduced pressure to obtain Compound **1f** (45 mg).

*¹*H NMR (400 MHz, CDCl₃) δ 7.06 (d, *J=* 8.7 Hz, 1H), 6.63 (d, *J =* 8.7 Hz, 1H), 5.19 (s, 1H), 3.87 (s, 3H), 3.55 (s, 2H), 2.33-2.28 (m, 4H), 1.9-1.79 (m, 2H).

Step 6) Compound **1f** (45.00 mg, 0.16 mmol) and Amberlyst^{®} 15 ion exchange resin (41.80 mg, 0.64 mmol) were added to toluene (5 mL) in a 25 mL single-neck flask at room temperature. The reaction system was stirred well, purged with nitrogen for 3 times, warmed to 90°C and stirred until completion of the reaction as detected by TLC. The reaction solution was filtered and concentrated under reduced pressure. The redidues were purified by column chromatography (ethyl acetate/petroleum ether) to obtain Compound **1g** (30 mg).

*¹H* NMR (400 MHz, DMSO-*d₆*) δ 6.93 (d, *J =* 8.7 Hz, 1H), 6.79 (d, *J =* 8.7 Hz, 1H), 3.74 (s, 3H), 3.16 (s, 2H), 2.04-1.90 (m, 2H), 1.86-1.66 (m, 6H).

Step 7) Compound **1g** (100.00 mg, 0.35 mmol), compound bis(pinacolato)diboron (179.36 mg, 0.71 mmol), potassium acetate (104.00 mg, 1.06 mmol) and [1,1'-bis(diphenylphosphino)ferrocene]palladium dichloride (25.80 mg, 0.035 mmol) were added to 1,4-dioxane (50 mL) in a 25 mL single-neck flask successively at room temperature. The reaction system was purged with nitrogen for 3 times, warmed to 90°C and stirred until completion of the reaction as detected by TLC, and filtered. Water (30 mL) was added and the reaction solution was extracted with ethyl acetate (50 mL × 3), dried over anhydrous sodium sulfate and concentrated under reduced pressure. The residues were purified by column chromatography (ethyl acetate/petroleum ether) to obtain Compound **1h** (45 mg).

*¹H* NMR (400 MHz, CDCl₃) δ 7.24 (d, *J=* 2.9 Hz, 1H), 6.72 (d, *J=* 8.1 Hz, 1H), 3.86 (s, 3H), 3.34 (s, 2H), 2.17-2.10 (m, 2H), 1.97-1.85 (m, 2H), 1.80-1.66 (m, 4H), 1.30 (s, 12H).

Step 8) Compound **1h** (45.00 mg, 0.14 mmol), compound 3-bromo-5-iodopyridine (38.69 mg, 0.14 mmol), potassium acetate (153.50 mg, 0.27 mmol) and [1,1'-bis(diphenylphosphino)ferrocene]palladium dichloride (41.00 mg, 0.014 mmol) were added to 1,4-dioxane and water (3:1, 8 mL) in a 25 mL single-neck flask successively at room temperature. The reaction system was purged with nitrogen for 3 times, warmed to 90°C and stirred until completion of the reaction as detected by TLC, and filtered. Water (20 mL) was added and the reaction solution was extracted with ethyl acetate (20 mL × 3), dried over anhydrous sodium sulfate and concentrated under reduced pressure. The residues were purified by column chromatography (ethyl acetate/petroleum ether) to obtain Compound **1i** (30 mg), LCMS: m/z 360.0 (M+H)⁺.

Step 9) Compound **1i** (100 mg, 0.28 mmol), Compound **1c** (176.02 mg, 0.42 mmol), potassium carbonate (76.75 mg, 0.56 mmol), potassium acetate (40.87 mg, 0.42 mmol) and [1,1'-bis(diphenylphosphino)ferrocene]palladium dichloride (20.05 mg, 0.023 mmol) were added to1,4-dioxane and water (3:1, 8 mL) in a 25 mL single-neck flask successively at room temperature. The reaction system was purged with nitrogen for 3 times, warmed to 90°C and stirred until completion of the reaction as detected by LCMS. The reaction solution was poured into water (20 mL), extracted with ethyl acetate (20 mL × 3), dried over anhydrous sodium sulfate, and concentrated under reduced pressure. The residues were purified by column chromatography (ethyl acetate/petroleum ether) to obtain Compound **1k** (60 mg).

LCMS: m/z 576.2 (M+H)⁺.

Step 10) Compound **1k** (60 mg, 0.10 mmol) and then a solution of hydrochloric acid in tetrahydrofuran (1:1, 4 mL) were added to a 25 mL single-neck flask at room temperature and stirred at 25°C. TLC detection showed that the reaction was completed. The reaction solution was concentrated under reduced pressure and purified by column chromatography (ethyl acetate/petroleum ether) to obtain Compound **1l** (40 mg).

LCMS: m/z 338.0 (M+H)⁺.

Step 11) Compound 11 (40 mg, 0.12 mmol) and then tetrahydrofuran (5 mL) were added to a 25 mL three-neck flask at room temperature and cooled to 0°C. A solution of borane in tetrahydrofuran (0.47 mL, 1 M, 0.48 mmol) was added dropwise. After addition, the reaction solution was naturally warmed to room temperature and stirred overnight. Water (0.5 mL) was added and continually stirred for 0.5 hour. LCMS detection showed that the reaction was complete. The reaction solution was purified by preparative liquid chromatography to obtain Compound 1 (1.31 mg).

LCMS: m/z 366.1 (M+H)⁺.

*¹H* NMR (400 MHz, MeOD) δ 8.40 (d, *J =* 26.6 Hz, 2H), 7.79 (s, 1H), 6.91 (q, *J =* 8.4 Hz, 2H), 4.58 (s, 3H), 4.17-3.95 (m, 1H), 3.89-3.85 (m, 3H), 3.83-3.78 (m, 1H), 2.16-2.01 (m, 2H), 1.93-1.88 (m, 2H), 1.84-1.68 (m, 5H), 1.37-1.22 (m, 1H), 1.19-1.09 (m, 1H).

The product was subjected to chiral resolution (Column: Chiralpak IG 5 µm 30 * 250 mm; Mobile Phase: Hex : EtOH = 35 : 65 at 15 mL/min; Temp: 30°C; Wavelength: 254 nm) to obtain Compound **1-1** (shorter retention time) and Compound **1-2** (longer retention time).

### Compound 1-1

LCMS: m/z 366.1 (M+H) ⁺.

¹*H* NMR (400 MHz, DMSO-*d*₆) δ 8.70 (s, 1H), 8.53 (d, *J =* 2.0 Hz, 1H), 8.43 (d, *J=* 1.9 Hz, 1H), 7.77 (s, 1H), 6.96-6.89 (m, 2H), 4.27 (t*, J =* 8.2 Hz, 1H), 3.83 (t, *J =* 8.9 Hz, 1H), 3.79 (s, 3H), 3.55-3.43 (m, 1H), 3.30 (s, 2H), 2.03-1.89 (m, 2H), 1.76-1.69 (m, 6H), 1.31 (dd, *J =* 16.2, 8.2 Hz, 1H), 1.11 (dd, *J=* 16.2, 10.2 Hz, 1H).

### Compound 1-2

LCMS: m/z 366.1 (M+H) ⁺.

*¹H* NMR (400 MHz, DMSO-*d₆*) δ 8.68 (s, 1H), 8.51 (d, *J =* 1.9 Hz, 1H), 8.41 (d, *J =* 1.7 Hz, 1H), 7.75 (s, 1H), 6.97-6.81 (m, 2H), 4.25 (t, *J =* 8.2 Hz, 1H), 3.81 (t, *J =* 8.9 Hz, 1H), 3.77 (s, 3H), 3.55-3.40 (m, 1H), 3.29-3.23 (m, 2H), 1.95-1.91 (m, 2H), 1.74-1.67 (m, 6H), 1.29 (dd, *J =* 16.1, 8.1 Hz, 1H), 1.09 (dd, *J =* 16.2, 10.2 Hz, 1H).

### Example 2

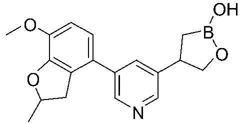

Compound **2** was synthesized according to the method of Example **1.**

LCMS: m/z 326.1 (M+H) ⁺.

*¹H* NMR (400 MHz, CD₃OD) δ 8.77 (s, 1H), 8.66 (s, 1H), 8.56 (s, 1H), 7.06-7.01 (m, 2H), 5.02 (d, *J =* 6.4 Hz, 1H), 3.90 (s, 3H), 3.88 (s, 2H), 3.47 (d, *J =* 8.5 Hz, 1H), 3.32 (s, 1H), 3.01 (dd, *J =* 15.4, 8.2 Hz, 1H), 1.48 (d, *J =* 6.2 Hz, 3H), 1.36 (m, 2H).

### Example 3

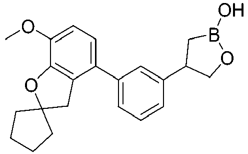

Compound 3 was synthesized according to the method of Example 1.

LCMS: m/z 365.1 (M+H) ⁺.

*¹H* NMR (400 MHz, DMSO-*d*₆) δ 8.63 (s, 1H), 7.37-7.26 (m, 3H), 7.21 (d, *J =* 7.4 Hz, 1H), 6.87 (dd, *J=* 19.4, 8.4 Hz, 2H), 4.28-4.20 (m, 1H), 3.82-3.74 (m, 4H), 3.51 -3.40 (m, 1H), 3.28 (s, 2H), 2.03-1.89 (m, 2H), 1.84-1.63 (m, 6H), 1.28 (dd, *J =* 16.2, 8.1 Hz, 1H), 1.05 (dd, *J =* 16.2, 9.8 Hz, 1H).

### Example 4

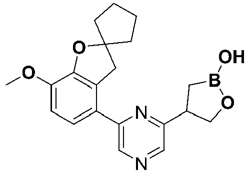

Compound **4** was synthesized according to the method of Example 1.

LCMS: m/z 367 (M+H) ⁺.

*¹H* NMR (400 MHz, DMSO-*d*₆) δ 8.86 (s, 1H), 8.64 (s, 1H), 8.44 (s, 1H), 7.41 (d, *J* = 8.4 Hz, 1H), 6.97 (d, *J =* 7.2 Hz, 1H), 4.31 (t, *J =* 10.8 Hz, 1H), 4.03-3.94 (m, 1H), 3.82 (s, 3H), 3.77-3.65 (m, 1H), 3.53 (d, *J =* 6.4 Hz, 2H), 1.97 (s, 2H), 1.78-1.75 (m, 6H), 1.32-1.30 (m, 1H), 1.22-1.12 (m, 1H).

### Example 5

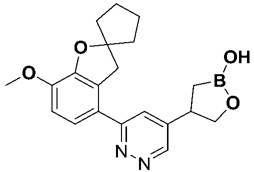

Compound 5 was synthesized according to the method of Example 1.

LCMS: m/z 367.2 (M+H) ⁺.

*¹H* NMR (400 MHz, DMSO-*d*₆) δ 9.10 (d, *J =* 2.0 Hz, 1H), 8.77 (s, 1H), 7.86 (d, *J =* 1.9 Hz, 1H), 7.34 (d, *J =* 8.5 Hz, 1H), 6.99 (d, *J =* 8.5 Hz, 1H), 4.33-4.27 (m, 1H), 3.89 (t, *J =* 8.9 Hz, 1H), 3.83 (s, 3H), 3.57-3.46 (m, 3H), 2.01-1.94 (m, 2H), 1.84-1.68 (m, 6H), 1.34 (dd, *J=* 16.3, 8.3 Hz, 1H), 1.16 (dd, *J =* 16.4, 10.4 Hz, 1H).

### Example 6

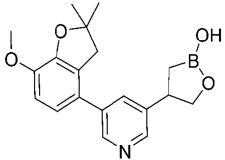

Compound 6 was synthesized according to the method of Example 1.

LCMS: m/z 340.1 (M+H) ⁺.

*¹H* NMR (400 MHz, DMSO-*d*₆) δ 8.70 (s, 1H), 8.52 (d, *J=* 2.1 Hz, 1H), 8.44 (d, *J =* 2.0 Hz, 1H), 7.77 (t, *J =* 2.0 Hz, 1H), 6.98-6.90 (m, 2H), 4.37-4.21 (m, 1H), 3.86-3.82 (m, 1H), 3.80 (s, 3H), 3.57-3.42 (m, 1H), 3.15 (s, 2H), 1.42 (s, 6H), 1.34-1.28 (m, 1H), 1.14-1.08 (m, 1H).

### Example 7

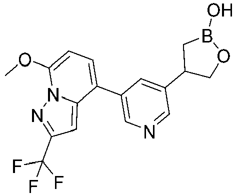

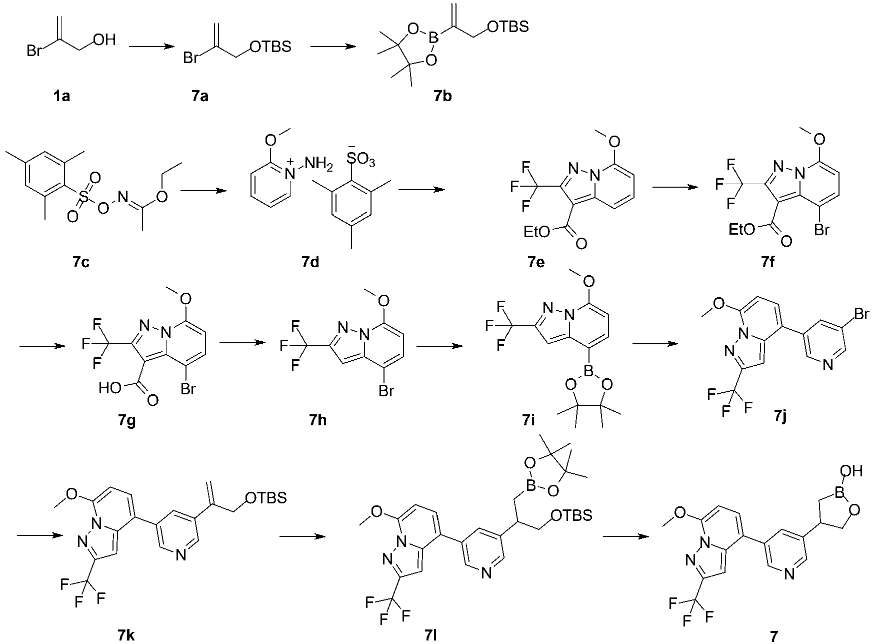

Step 1) Compound **1a** (15.00 g, 109.51 mmol) and imidazole (6.71 g, 98.555 mmol) were dissolved in *N,N-dimethyl* sulfoxide (100 mL). Tert-butyldimethylchlorosilane (14.03 g, 93.08 mmol) was added at 30°C for reaction for 3 hours at room temperature. TLC detection showed that the reaction was complete. The reaction solution was added into water (100 mL) to quench the reaction, which was then extracted with methyl tert-butyl ether (100 mL × 3) and dried over anhydrous sodium sulfate to obtain Compound **7a** (25.00 g).

*¹H* NMR (400 MHz, CDCl₃) δ 5.98 (dd, *J =* 3.5, 1.7 Hz, 1H), 5.55 (dd, *J=* 3.1, 1.5 Hz, 1H), 4.23 (t, *J =* 1.7 Hz, 2H), 0.95 (s, 9H), 0.12 (s, 6H).

Step 2) Compound **7a** (25.00 g, 99.51 mmol), bis(pinacolato)diboron (27.80 g, 109.46 mmol), potassium acetate (19.53 g, 199.01 mmol) and bis(triphenylphosphino) palladium dichloride (1.55 g, 1.99 mmol) were dissolved in 1,4-dioxane (90 mL) at room temperature. The reaction system was purged with nitrogen for 3 times and then stirred at 80°C for 16 hours. LCMS detection showed that the reaction was complete. Water (200 mL) and ethyl acetate (100 mL×3) was added to the reaction solution for extraction. The organic phase was dried over anhydrous sodium sulfate and concentrated. The residues were purified by column chromatography (ethyl acetate/petroleum ether) to obtain the product **7b** (11.00 g).

*¹H* NMR (400 MHz, DMSO-*d*₆) δ 5.85-5.80 (m, 1H), 5.73-5.71 (m, 1H), 4.17 (t, *J =* 1.9 Hz, 2H), 1.20 (s, 12H), 0.87 (s, 9H), 0.03 (s, 6H).

Step 3) Compound **7c** (10.0 g, 35.04 mmol) was dissolved in 1,4-dioxane (14 mL) and cooled to 0°C. Perchloric acid (5.14 mL, 59.57 mmol, 70% wt.) was added dropwise and stirred at 0°C for 0.5 hour. Ice water (140 mL) was added and white solids were precipitated and filtered. The white solids were dissolved in dichloromethane (200 mL) and the aqueous phase was separated. The organic phase was dried over anhydrous sodium sulfate and filtered. The filtrate was added dropwise to a solution of compound 2-methoxy-pyridine (5.93 g, 54.32 mmol) in dichloromethane (100 mL) at 0°C. After the addition, the reaction was carried out at room temperature for 1 hour. TLC detection showed that the reaction was complete. The reaction solution was concentrated under reduced pressure. The residues were added to anhydrous ether and white solids were precipitated and filtered to obtain the target Compound **7d** (9.5 g).

*¹H* NMR (400 MHz, DMSO-*d*₆) δ 8.55 (dd, *J =* 6.5, 1.5 Hz, 1H), 8.28-8.24 (m, 1H), 7.72 (dd, *J =* 6.6, 3.1 Hz, 1H), 7.53-7.45 (m, 1H), 6.74 (s, 2H), 4.26 (s, 3H), 2.50 (dd, *J=* 4.0, 2.1 Hz, 6H), 2.17 (s, 3H).

Step 4) Compound **7d** (9.0 g, 27.74 mmol) and ethyl 4,4,4-trifluoro-2-butynoate (4.61 g, 27.74 mmol) were dissolved in *N,N-*dimethylformamide (20 mL). Potassium carbonate (7.7 g, 55.48 mmol) was added and stirred at room temperature for 16 hours. LCMS detection showed that the reaction was complete. The reaction solution was concentrated under reduced pressure. The residues were purified by column chromatography (ethyl acetate/petroleum ether) to obtain Compound **7e** (3.05 g).

LCMS: m/z 289.0 (M+H) ⁺.

Step 5) Compound **7e** (2.9 g, 10.06 mmol) was dissolved in acetonitrile (20 mL) at room temperature. *N*-bromosuccinimide (2.7 g, 15.09 mmol) was added. The reaction solution was purged with nitrogen for 3 times and warmed to 70°C for reaction for 5 hours. LCMS detection showed that the reaction was complete. The reaction solution was concentrated under reduced pressure. The residues were purified by column chromatography (ethyl acetate/petroleum ether) to obtain Compound **7f** (1.5 g).

LCMS: m/z 366.9 (M+1) ⁺.

Step 6) Compound **7f** (1.5 g, 4.09 mmol) was dissolved in methanol (10 mL). A solution of potassium hydroxide (917 mg, 16.34 mmol) in water (5 mL) was added dropwise for reaction at room temperature for 16 hours. LCMS detection showed that the reaction was complete. 1 N hydrochloric acid was added to adjust the pH to ~7. The reaction solution was concentrated under reduced pressure to remove the methanol, and then added with 1 N hydrochloric acid to adjust the pH to ~2. The solution was extracted with ethyl acetate (50 mL × 3), and washed with water (25 mL × 3) and saturated sodium chloride aqueous solution. The organic phase was dried over anhydrous sodium sulfate, filtered and concentrated under reduced pressure to obtain Compound **7g** (1.3 g).

LCMS: m/z 339.9 (M+H) ⁺.

Step 7) Compound **7g** (1.3 g, 3.83 mmol) was dissolved in ethanol (20 mL). Concentrated sulfuric acid (1 mL) was added. The reaction solution was warmed to 90°C for reaction for 16 hours. LCMS detection showed that the reaction was complete. The reaction solution was concentrated under reduced pressure. A saturated solution of sodium bicarbonate was added to adjust the pH to ~9. The reaction solution was extracted with ethyl acetate (50 mL × 3) and washed with water (25 mL × 3) and saturated s sodium chloride aqueous solution (25 mL × 3). The organic phase was dried over anhydrous sodium sulfate, filtered and concentrated under reduced pressure to obtain Compound 7h (749 mg).

LCMS: m/z 356.2 (M+H) ⁺.

Step 8) Compound 7h (300 mg, 1.02 mmol), bis(pinacolato)diboron (387 mg, 1.53 mmol), [1,1'-bis(diphenylphosphino)ferrocene]palladium dichloride (75 mg, 0.102 mmol) and potassium acetate (19.53 g, 199.01 mmol) were mixed in 1,4-dioxane (5 mL). The reaction solution was purged with nitrogen for 3 times and warmed to 100°C for reaction for 16 hours. LCMS detection showed that the reaction was complete. The reaction solution was concentrated under reduced pressure. The residues were purified by column chromatography (ethyl acetate/petroleum ether) to obtain Compound **7i** (300 mg).

LCMS: m/z 343.1 (M+H) ⁺.

Step 9) Compound **7i** (0.9 g, 2.63 mmol), 3,5-dibromopyridine (0.612 mL, 5.26 mmol), potassium carbonate (0.55 g, 3.95 mmol) and [1,1'-bis(diphenylphosphino)ferrocene]palladium dichloride (0.10 g, 0.13 mmol) were mixed in 1,4-dioxane/water (10 mL/0.2 mL). The reaction solution was purged with nitrogen for 3 times and stirred at 90°C for 16 hours. LCMS detection showed that the reaction was complete. The reaction solution was filtered and concentrated under reduced pressure. The residues were purified by column chromatography (ethyl acetate/petroleum ether) to obtain Compound **7j** (850 mg).

LCMS: m/z 374 (M+H) ⁺.

Step 10) Compound **7j** (800 mg, 2.15 mmol), Compound **6b** (833.67 mg, 2.79 mmol), potassium acetate (316.46 mg, 3.23 mmol), potassium carbonate (594.23 mg, 4.3 mmol) and [1,1'-bis(diphenylphosphino)ferrocene]palladium dichloride (78.65 mg, 0.11 mmol) were mixed in 1,4-dioxane/water (10 mL/0.02 mL) at room temperature. The reaction solution was purged with nitrogen for 3 times and stirred at 90°C for 16 hours. LCMS detection showed that the reaction was complete. The reaction solution was filtered and concentrated under reduced pressure. The residues were purified by silica gel column chromatography (ethyl acetate/petroleum ether) to obtain Compound **7k** (430 mg).

LCMS: m/z 464 (M+H) ⁺.

Step 11) 1,2-Bis(diphenylphosphino)ethane (32.67 mg, 0.082 mmol) and (1,5-cyclooctadienyl)methoxyiridium (I) dimer (27.17 mg, 0.041 mmol) were added to 1,2-dichloroethane (3 mL) in a thoroughly dried 100 mL three-neck flask equipped with a thermometer under nitrogen atmosphere at room temperature, and stirred at room temperature for 15 min. Compound **7k** (190 mg, 0.410 mmol) was added and stirred at room temperature for 15 min. The mixed solution was stirred and heated in an oil bath at 95°C, and pinacolborane (0.416 mL, 2.87 mmol) was added dropwise when the reading of the thermometer reached 70°C. The reaction solution was stirred for reaction for 0.5 h. LCMS detection showed that the reaction was complete. The reaction solution was let stand until cooled to room temperature and 5 mL of methanol was added to quench the reaction. The reaction solution was concentrated under reduced pressure and purified by silica gel column chromatography (ethyl acetate/petroleum ether) to obtain Compound **71** (120 mg).

LCMS: m/z 592 (M+H) ⁺.

Step 12) Compound **71** (100 mg, 0.17 mmol) was dissolved in tetrahydrofuran (1 mL) at room temperature. 1 N hydrochloric acid (1 mL) was slowly added dropwise under stirring for reaction at room temperature for 0.5 hour. LCMS detection showed that the reaction was complete. The reaction solution was concentrated and diluted with ethyl acetate (5 mL). A saturated potassium phosphate aqueous solution was added to adjust the pH to around 8. The reaction solution was extracted with ethyl acetate (10 mL × 3). The organic phase was dried over anhydrous sodium sulfate, concentrated and purified by preparative high performance liquid chromatography (Pre-HPLC) to obtain Compound **7** (5.54 mg).

LCMS: m/z 378 (M+1) ⁺.

*¹H* NMR (400 MHz, DMSO-*d*₆) δ 8.72 (d, *J =* 2.0 Hz, 1H), 8.70 (s, 1H), 8.57 (m, 1H), 7.99 (s, 1H), 7.62 (d, *J =* 7.6 Hz, 1H), 7.19 (s, 1H), 6.78 (d, *J =* 8.0 Hz, 1H), 4.34-4.28 (t, *J =* 8.6 Hz, 1H), 4.20 (s, 3H), 3.89 (t, *J =* 8.8 Hz, 1H), 3.62-3.51 (m, 1H), 1.34 (m, 1H), 1.19-1.12 (m, 1H).

### Example 8

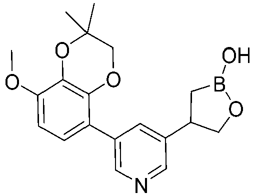

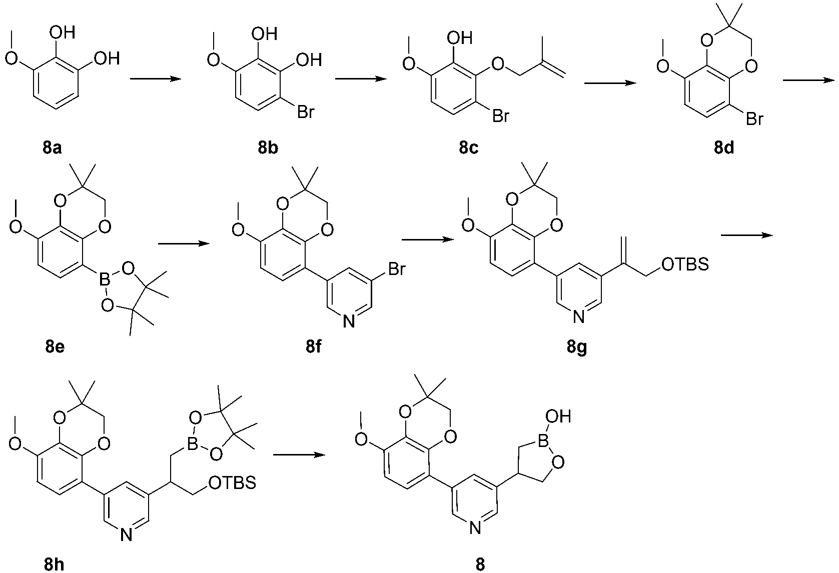

Step 1) Compound **8a** (25.00 g, 178.39 mmol) and N-bromosuccinimide (31.75 g, 178.39 mmol) were added to acetonitrile (200 mL) in a 500 mL single-neck flask at room temperature, and stirred until dissolved. The reaction system was purged with nitrogen for 3 times and stirred at room temperature for 16 hours. TLC detection showed that the reaction was completed. The reaction solution was concentrated, diluted with ethyl acetate (100 mL) and poured into a sodium bisulfite aqueous solution (500 mL). The pH was adjusted to around 3. The reaction solution was extracted with ethyl acetate (200 mL). The organic phase was dried over anhydrous sodium sulfate, filtered and concentrated. The residues were purified by column chromatography (ethyl acetate/petroleum ether) to obtain Compound **8b** (24.00 g).

*¹H* NMR (400 MHz, DMSO-*d*₆) δ 9.12 (s, 1H), 8.94 (s, 1H), 6.87 (d, *J =* 8.9 Hz, 1H), 6.45 (d, *J =* 8.9 Hz, 1H), 3.76 (s, 3H).

Step 2) Compound **8b** (100.00 mg, 0.46 mmol), 3-bromo-2-methylpropene (61.64 mg, 0.46 mmol) and potassium carbonate (94.65 mg, 0.69 mmol) were added to *N,N-*dimethylformamide (10 mL) in a 100 mL single-neck flask into at room temperature. The reaction system was purged with nitrogen for 3 times and stirred at room temperature for 16 hours. TLC detection showed that the reaction was completed. The reaction solution was filtered and concentrated under reduced pressure. A saturated sodium chloride aqueous solution (100 mL) was added. The reaction solution was extracted with ethyl acetate (100 mL). The organic phase was dried over anhydrous sodium sulfate, filtered, and concentrated under reduced pressure. The residues were purified by column chromatography (ethyl acetate/petroleum ether) to obtain Compound 8c (25.00 mg).

*¹H* NMR (400 MHz, DMSO-*d*₆) δ 9.03 (s, 1H), 6.94 (d, *J =* 8.8 Hz, 1H), 6.67 (d, *J =* 8.9 Hz, 1H), 5.07 (s, 1H), 4.91 (s, 1H), 4.36 (s, 2H), 3.76 (s, 3H), 1.82 (s, 3H).

Step 3) Compound **8c** (50.00 mg, 0.18 mmol) and iodine (9.29 mg, 0.037 mmol) were added to dichloromethane (2 mL) in a 100 mL single-mouth flask at room temperature and stirred until dissolved. The reaction system was purged with nitrogen for 3 times and stirred at room temperature for 16 hours. TLC detection showed that the reaction was completed. The reaction solution was added to a sodium thiosulfate saturated solution (25 mL), and extracted with ethyl acetate (30 mL × 3). The organic phase was dried over anhydrous sodium sulfate and concentrated. The residues were purified by column chromatography (ethyl acetate/petroleum ether) to obtain Compound **8d** (20.0 mg).

*¹H* NMR (400 MHz, DMSO-*d*₆) δ 7.83 (d, *J =* 8.9 Hz, 1H), 7.37 (d, *J =* 8.9 Hz, 1H), 4.80 (s, 2H), 4.53 (s, 3H), 2.08 (s, 6H).

Step 4) Compound **8d** (400.00 mg, 1.46 mmol), Biboric acid (557.84 mg, 2.2 mmol), potassium acetate (431.18 mg, 4.39 mmol) and 1,1-bis(diphenyl)ferrocene palladium dichloride (53.60 mg, 0.073 mmol) were added to 1,4-dioxane (10 mL) in a 50 mL single-neck flask at room temperature, and stirred until dissolved. The reaction system was purged with nitrogen for 3 times and stirred at 110°C for 16 hours. LCMS detection showed that the reaction was completed. The reaction solution was added to water (100 mL) and extracted with ethyl acetate (100 mL × 3). The organic phase was dried over anhydrous sodium sulfate and concentrated. The residues were purified by column chromatography (ethyl acetate/petroleum ether) to obtain Compound 8e (0.26 g).

LCMS: m/z 321.1 (M+H) ⁺.

Step 5) Compound **8e** (240.00 mg, 0.750 mmol), 3,5-dibromopyridine (355.12 mg, 1.5 mmol), potassium carbonate (207.18 mg, 1.5 mmol) and 1,1-bis(diphenyl)ferrocene palladium dichloride (54.87 mg, 0.075 mmol) were added to a mixed solvent of 1,4-dioxane (6 mL) and water (2 mL) in a 25 mL single-neck flask at room temperature, and stirred until dissolved. The reaction system was purged with nitrogen for 3 times and stirred at 90°C for 16 hours. LCMS detection showed that the reaction was completed. The reaction solution was added to water (100 mL) and extracted with ethyl acetate (100 mL × 3). The organic phase was dried and then concentrated. The residues were purified by column chromatography (ethyl acetate/petroleum ether) to obtain Compound **8f** (150.00 mg).

LCMS: m/z 351.9 (M+H+2) ⁺.

Step 6) Compound **8f** (130.00 mg, 0.37 mmol), Compound **7b** (143.95 mg, 0.48 mmol), potassium carbonate (102.60 mg, 0.74 mmol), potassium acetate (54.65 mg, 0.56 mmol) and 1,1-bis(diphenyl)ferrocene palladium dichloride (13.59 mg, 0.019 mmol) were added to a mixed solvent of 1,4-dioxane (6 mL) and water (0.12 mL) in a 100 mL single-neck flask at room temperature, and stirred until dissolved. The reaction system was purged with nitrogen for 3 times and stirred at 110°C for 16 hours. LCMS detection showed that the reaction was completed. The reaction solution was added to 100 mL of water and extracted with ethyl acetate (200 mL × 3). The organic phase was dried and then concentrated. The residues were purified by column chromatography (ethyl acetate/petroleum ether) to obtain Compound **8g** (150.00 mg).

LCMS: m/z 442.1 (M+H) ⁺.

Step 7) 1,2-Bis(diphenylphosphino)ethane (23.46 mg, 0.059 mmol) and (1,5-cyclooctadienyl)methoxyiridium (I) dimer (19.51 mg, 0.029 mmol) were added to a 50 mL three-neck flask at room temperature. Then anhydrous 1,2-dichloroethane (15 mL) was added. Compound **8g** (130.00 mg, 0.294 mmol, dissolved in 5 mL of 1,2-dichloroethane) was slowly added and stirred at room temperature for 15 minutes. Then the reaction solution was warmed to 70°C and pinacolborane (263.70 mg, 2.061 mmol) was added dropwise. After the addition, the reaction solution was stirred at 70°C for 1 hour. LCMS detection showed that the reaction was completed. The reaction solution was cooled to 0 °C. 20 mL of methanol was added dropwise to quench the reaction. Then the reaction solution was concentrated and subjected to column chromatography (ethyl acetate/petroleum ether) to obtain Compound **8h** (120.00 mg).

LCMS: m/z 570.1 (M+H) ⁺.

Step 8) The reactant **8h** (100.00 mg, 0.17 mmol) and the solvent tetrahydrofuran (2 mL) were added to a 25 mL of eggplant type flask at room temperature and cooled to 0°C. 1 N hydrochloric acid (2 mL) was added dropwise. After the addition, the reaction solution was stirred at room temperature for 1 hour. LCMS detection showed that the reaction was completed. The reaction solution was concentrated under reduced pressure and extracted with ethyl acetate (100 mL × 3). A saturated aqueous solution of potassium phosphate was added to the aqueous phase to adjust the pH to = 8.0. The reaction solution was extracted with ethyl acetate (100 mL × 3). The organic phases were pooled, washed with water (100 mL × 3) and saturated saline (100 mL × 3). The organic phase was dried over anhydrous sodium sulfate, filtered and concentrated under reduced pressure. Then the residues were purified by preparative liquid chromatography (Pre-HPLC) to obtain Compound **8** (5.46 mg).

LCMS: m/z 356.1 (M+H) ⁺.

*¹H* NMR (400 MHz, DMSO-*d*₆) δ 8.69 (s, 1H), 8.53 (s, 1H), 8.39 (s, 1H), 7.75 (s, 1H), 6.87 (d, *J =* 8.4 Hz, 1H), 6.70 (d, *J =* 8.5 Hz, 1H), 4.27 (t, *J =* 8.1 Hz, 1H), 3.92 (s, 2H), 3.82 (t, *J=* 8.9 Hz, 1H), 3.78 (s, 3H), 3.51-3.46 (m, 1H), 1.34-1.28 (m, 7H), 1.14-1.07(m, 1H).

### Example 9

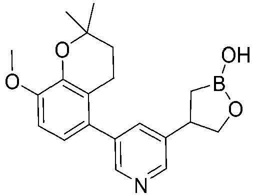

Compound 9 was synthesized according to the method of Example **8.**

LCMS: m/z 354.2 (M+H) ⁺.

*¹H* NMR (400 MHz, DMSO-*d*₆) δ 8.70 (s, 1H), 8.44 (d, *J =* 1.8 Hz, 1H), 8.38 (d, *J =* 1.8 Hz, 1H), 7.69 (s, 1H), 6.87 (d, *J =* 8.3 Hz, 1H), 6.71 (d, *J =* 8.3 Hz, 1H), 4.27 (t, *J =* 8.2 Hz, 1H), 3.82 (t, *J =* 8.9 Hz, 1H), 3.75 (s, 3H), 3.55-3.42 (m, 1H), 2.54-2.50 (m, 2H), 1.66 (t, *J =* 6.5 Hz, 2H), 1.38-1.23 (m, 7H), 1.17-0.97 (m, 1H).

### Example 10

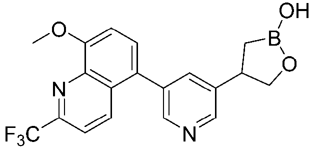

Compound **10** was synthesized according to the method of Example **8.**

LCMS: m/z 389.1 (M+H) ⁺.

*¹H* NMR (400 MHz, DMSO-*d*₆) δ 8.71 (s, 1H), 8.61 (d, *J =* 1.5 Hz, 1H), 8.55 (d, *J =* 1.6 Hz, 1H), 8.42 (d, *J =* 8.8 Hz, 1H), 7.99 (d, *J =* 8.9 Hz, 1H), 7.87-7.84 (m, 1H), 7.74 (d, *J =* 8.1 Hz, 1H), 7.46 (d, *J=* 8.2 Hz, 1H), 4.31 (t, *J =* 8.3 Hz, 1H), 4.08 (s, 3H), 3.88 (t, *J* = 8.9 Hz, 1H), 3.58-3.55 (m, 1H), 1.34 (dd, *J =* 16.2, 8.1 Hz, 1H), 1.15 (dd*, J =* 16.2, 10.4 Hz, 1H).

### Example 11

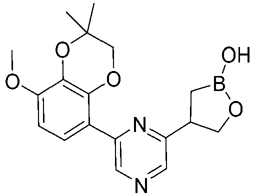

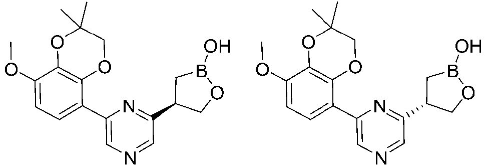

### (Compounds 11-1 and 11-2)

Compound **11** was synthesized according to the method of Example **9** and subjected to chiral resolution to obtain Compound 11**-1** (shorter retention time) and Compound **11-**2 (longer retention time).

### Compound 11-1

LCMS: m/z 357.2 (M+H) ⁺.

*¹H* NMR (400 MHz, DMSO-*d*₆) δ 8.93 (s, 1H), 8.64 (s, 1H), 8.42 (s, 1H), 7.37 (d, *J* = 8.8 Hz, 1H), 6.76 (d, *J =* 8.8 Hz, 1H), 4.34-4.26 (m, 1H), 4.07-3.97 (m, 3H), 3.80 (s, 3H), 3.73-3.62 (m, 1H), 1.36-1.27 (m, 7H), 1.25-1.14 (m, 1H).

### Compound 11-2

LCMS: m/z 357.2 (M+H) ⁺.

*¹H* NMR (400 MHz, DMSO-*d*₆) δ 8.93 (s, 1H), 8.64 (s, 1H), 8.42 (s, 1H), 7.37 (d, *J* = 8.8 Hz, 1H), 6.76 (d, *J =* 8.8 Hz, 1H), 4.39-4.23 (m, 1H), 4.06-3.93 (m, 3H), 3.80 (s, 3H), 3.73-3.62 (m, 1H), 1.33-1.23 (m, 7H), 1.21-1.15 (m, 1H).

### Example 12

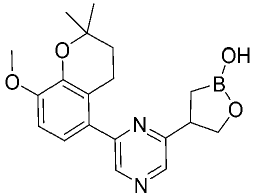

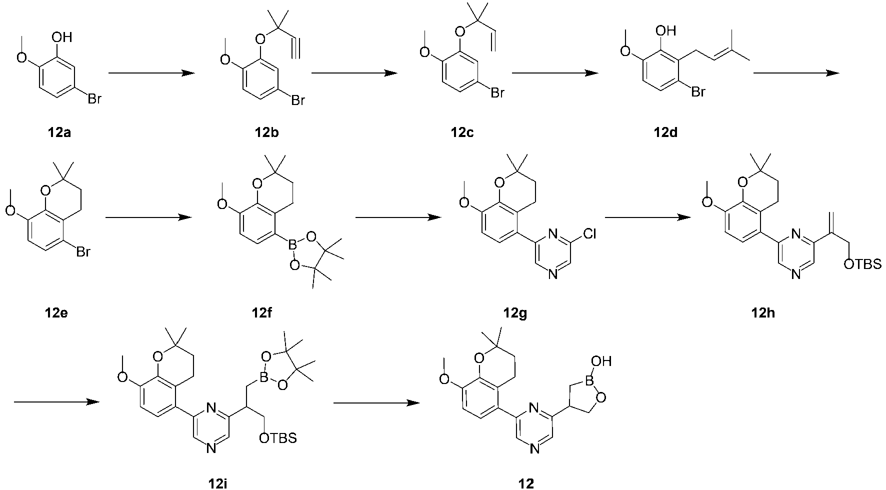

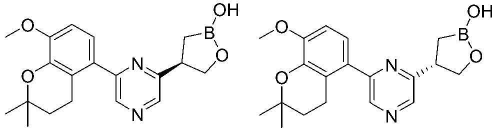

### (Compounds 12-1 and 12-2)

Step 1) Compound **12a** (100 g, 492 mmol), DMF (1000 mL), potassium iodide (92.6 g, 837 mmol), cuprous iodide (3.13 g, 9.85 mmol) and potassium carbonate (136 g, 985 mmol) were added to a 2000 mL single-neck flask successively at room temperature. Compound 3-chloro-3-methyl-1-butyne (100 mL, 886 mmol) was added dropwise under nitrogen atmosphere and stirred at 70°C for 16 hours. The reaction solution was cooled to room temperature, and added to water (1000 mL), which was then extracted with petroleum ether (1000 mL × 3). The organic phase was dried over anhydrous sodium sulfate, filtered and concentrated under reduced pressure. The residues were purified by column chromatography (petroleum ether/ethyl acetate) to obtain Compound **12b** (50 g).

*¹H* NMR (400 MHz, CDCl₃) δ 7.57 (d, *J =* 2.4 Hz, 1H), 7.15 (dd, *J =* 8.8, 2.4 Hz, 1H), 6.76 (d, *J=* 8.8 Hz, 1H), 3.79 (s, 3H), 2.58 (s, 1H), 1.65 (s, 6H).

Step 2) Compound **12b** (20.0 g, 74.4 mmol), n-hexane (200 mL) and palladium calcium carbonate (1.95 g, 18.8 mmol) were added to a 500 mL single-neck flask successively at room temperature. The reaction solution was stirred at room temperature under hydrogen atmosphere for 16 hours, filtered and concentrated under reduced pressure to obtain Compound **12c** (19 g).

*¹H* NMR (400 MHz, CDCl₃) δ 7.15 (d, *J =* 2.4 Hz, 1H), 7.09 (dd, *J =* 8.8, 2.4 Hz, 1H), 6.73 (d, *J =* 8.8 Hz, 1H), 6.12 (dd, *J =* 17.6, 10.8 Hz, 1H), 5.14 (dd, *J =* 20.0, 9.2 Hz, 2H), 3.79 (s, 3H), 1.46 (s, 6H).

Step 3) Compound **12c** (10.0 g, 36.9 mmol) and diethylaniline (10 mL, 62.5 mmol) were added to a 50 mL single-neck flask successively at room temperature. The reaction solution was stirred at 210°C for 1 hour and cooled to room temperature. 1 M HCl was added to adjust the pH to neutral. The reaction solution was extracted with ethyl acetate (200 mL × 3), washed with water (200 mL × 3) and concentrated under reduced pressure to obtain Compound **12d** (9.1 g).

*¹H* NMR (400 MHz, DMSO-*d*₆) δ 8.97 (s, 1H), 6.97 (d, *J =* 8.8 Hz, 1H), 6.77 (d, *J=* 8.8 Hz, 1H), 5.14-5.01 (m, 1H), 3.78 (s, 3H), 3.40 (d, *J =* 6.8 Hz, 2H), 1.74 (s, 3H), 1.63 (s, 3H).

Step 4) Compound **12d** (5.00 g, 18.5 mmol), toluene (25 mL) and Amberlyst^{®} 15 (5.00 g, 15.9 mmol) were added to a 100 mL single-neck flask successively at room temperature. The reaction solution was stirred at 100°C under nitrogen atmosphere for 2 hours and then cooled to room temperature. The reaction mixture was filtered and concentrated under reduced pressure to obtain Compound **12e** (3.89 g).

*¹H* NMR (400 MHz, DMSO-*d*₆) δ 7.04 (d, *J =* 8.8 Hz, 1H), 6.75 (d, *J =* 8.8 Hz, 1H), 3.71 (s, 3H), 2.63 (t*, J =* 6.8 Hz, 2H), 1.78 (t, *J =* 6.8 Hz, 2H), 1.26 (s, 6H).

Step 5) Compound **12e** (28.4 g, 105 mmol), dioxane (300 mL), bis(pinacolato)diboron (31.9 g, 126 mmol), potassium acetate (20.6 g, 209 mmol) and [1,1'-bis(diphenylphosphino)ferrocene]palladium dichloride (7.66 g, 10.5 mmol) were added to a 500 mL single-neck flask successively at room temperature. The reaction solution was stirred at 105°C under nitrogen atmosphere for 16 hours, and then cooled to room temperature. The reaction mixture was filtered and concentrated under reduced pressure. The residues were purified by column chromatography (petroleum ether/ethyl acetate) to obtain Compound **12f** (28.4 g).

LCMS: m/z 319 (M+H) ⁺.

Step 6) Compound **12f** (6.20 g, 19.5 mmol), 1,4-dioxane (80 mL), water (16 mL), 2,6-dichloropyrazine (2.90 g, 39.0 mmol), potassium carbonate (5.39 g, 39.0 mmol) and [1,1'-bis(diphenylphosphino)ferrocene]palladium dichloride (1.43 g, 1.95 mmol) were added to a 250 mL single-neck flask successively at room temperature. The reaction solution was stirred at 110°C under nitrogen atmosphere for 16 hours, and then cooled to room temperature. The reaction mixture was filtered and concentrated under reduced pressure. The residues were purified by column chromatography (petroleum ether/ethyl acetate) to obtain Compound **12g** (5.7 g).

LCMS: m/z 305 (M+H) ⁺.

Step 7) Compound **12g** (35.0 g, 115 mmol), Compound **1c** (44.5 g, 149 mmol), potassium carbonate (23.8 g, 172 mmol), potassium acetate (16.9 g, 172 mmol) and [1,1'-bis(diphenylphosphino)ferrocene]palladium dichloride (8.41 g, 11.5 mmol) were added to a mixed solvent of 1,4-dioxane (250 mL) and water (5 mL) in a 500 mL single-neck flask at room temperature, and stirred until dissolved. The reaction system was purged with nitrogen for 3 times, and stirred 110°C for 16 hours. The reaction solution was cooled to room temperature. Water (500 mL) was added and the reaction solution was extracted with ethyl acetate (300 mL × 3), dried over anhydrous sodium sulfate and concentrated. The residues were purified by column chromatography (petroleum ether/ethyl acetate) to obtain Compound **12h** (37 g).

LCMS: m/z 441.1 (M+H) ⁺.

Step 8) 1,2-Bis(diphenylphosphino)ethane (1.81 g, 4.54 mmol), (1,5-cyclooctadienyl)methoxyiridium (I) dimer (1.50 g, 2.27 mmol) and anhydrous 1,2-dichloroethane (100 mL) were added to a 250 mL three-neck flask at room temperature, and stirred at room temperature for 10 minutes. Compound **12h** (10.0 g, 22.7 mmol) was added. The reaction solution was warmed to 70°C and pinacolborane (20.3 g, 159 mmol) was added dropwise. The reaction was carried out at 95°C for 4 hours. The reaction solution was cooled to 0°C. Methanol (50 mL) was added dropwise to quench the reaction. The reaction solution was concentrated. The residues were purified by column chromatography (petroleum ether/ethyl acetate) to obtain Compound **12i** (1.4 g).

LCMS: m/z 569.3 (M+H) ⁺.

Step 9) The reactant **12i** (9.00 g, 15.8 mmol) and tetrahydrofuran (30 mL) were added to a 50 mL single-neck flask at room temperature and cooled to 0°C. 2 M hydrochloric acid (50 mL) was added and stirred at 50°C for 16 hours. The reaction solution was concentrated under reduced pressure and extracted with ethyl acetate (300 mL × 3). The organic phase was dried over anhydrous sodium sulfate, filtered and concentrated under reduced pressure. The residues were purified by reversed-phase column chromatography (acetonitrile/water/trifluoroacetic acid) to obtain Compound **12** (3.82 g).

The product was subjected to chiral resolution (Column: Chiralpak IG 5 µm 30 * 250 mm; Mobile Phase: Hex: EtOH = 35: 65 at 15 mL/min; Temp: 30°C; Wavelength: 254 nm) to obtain Compound **12-1** (shorter retention time) and Compound **12-2** (longer retention time).

### Compound 12-1

LCMS: m/z 355.0 (M+H) ⁺.

*¹H* NMR (400 MHz, DMSO-*d*₆) δ 8.62 (s, 1H), 8.48 (s, 1H), 6.98 (d, *J =* 8.4 Hz, 1H), 6.90 *(d, J =* 8.4 Hz, 1H), 4.29 (dd, *J =* 8.8, 7.6 Hz, 1H), 3.96 (dd*, J =* 8.8, 6.8 Hz, 1H), 3.77 (s, 3H), 3.74-3.62 (m, 1H), 2.93-2.67 (m, 2H), 1.68 (t, *J =* 6.8 Hz, 2H), 1.38-1.24 (m, 7H), 1.21-1.08 (m, 1H).

### Compound 12-2

LCMS: m/z 355.0 (M+H) ⁺.

*¹H* NMR (400 MHz, DMSO-*d*₆) δ 8.62 (s, 1H), 8.48 (s, 1H), 6.98 (d, *J =* 8.4 Hz, 1H), 6.90 (d, *J* = 8.4 Hz, 1H), 4.29 (dd, *J =* 8.8, 7.6 Hz, 1H), 3.96 (dd, *J =* 8.8, 6.8 Hz, 1H), 3.77 (s, 3H), 3.74-3.60 (m, 1H), 2.89-2.67 (m, 2H), 1.68 (t, *J =* 6.8 Hz, 2H), 1.36-1.25 (m, 7H), 1.21-1.07 (m, 1H).

### Example 13

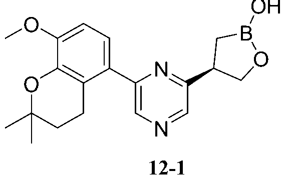

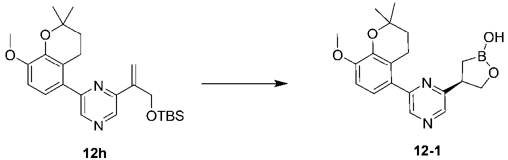

Compound **12h** (400 mg, 0.91 mmol) was dissolved in anhydrous THF (4 mL) at room temperature. The reaction solution was cooled to -10°C under N₂ atmosphere. 1,5-Cyclooctadiene iridium chloride dimer (21 mg, 0.041 mmol, 4.5% mol) and (S)-1-(diphenylphosphino)-2-[(*S*)-4-isopropyloxazolin-2-yl]ferrocene (52 mg, 0.11 mmol, 12%mol) were added. The reaction solution was stirred at room temperature for 15 min. A solution of catecholborane in THF (1 M, 7.2 mL, 7.2 mmol) was added and the reaction was continued at room temperature for 3 hours. Concentrated hydrochloric acid (0.2 mL) was added and the reaction was continued for 2 hours. The reaction solution was filtered and concentrated under reduced pressure. The residues were purified by reversed-phase column chromatography (acetonitrile/water/trifluoroacetic acid) to obtain a compound (105 mg), which was identified by chiral HPLC to obtain a compound as same as Compound **12-1** obtained by resolution.

LCMS: m/z 355.0 (M+H) ⁺.

*¹H* NMR (400 MHz, DMSO-*d*₆) δ 8.62 (s, 1H), 8.48 (s, 1H), 6.98 (d, *J =* 8.4 Hz, 1H), 6.90 (d, *J =* 8.4 Hz, 1H), 4.29 (dd, *J =* 8.8*,* 7.6 Hz, 1H), 3.96 (dd, *J =* 8.8, 6.8 Hz, 1H), 3.77 (s, 3H), 3.74-3.62 (m, 1H), 2.93-2.67 (m, 2H), 1.68 (t, *J =* 6.8 Hz, 2H), 1.38-1.24 (m, 7H), 1.21-1.08 (m, 1H).

### BIOLOGICAL EVALUATION

The present disclosure is further described and explained below with reference to the test examples, but these test examples are not intend to limit the scope of the present disclosure.

The structure of Compound **A** is

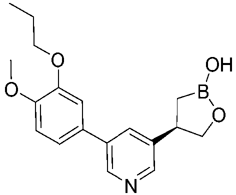

Compound **A** was prepared by the method disclosed in Example 4 on page 181 of the specification of the patent application WO2020070651A.

### Test Example 1 In vitro PDE4B enzyme activity detection test

### 1. Experimental materials

| Name | Brand | Cat No. |
|---|---|---|
| PDE4B1 enzyme | BPS | 60041 |
| Trequinsin | TOCRIS | 2337/10 |
| IMAP FP IPP Explorer Kit | Molecular Device | R8124 |
| FAM-cAMP | Molecular Device | R7506 |
| OptiPlate^{™}-384 F black assay plate | PerkinElmer | 6007279 |
| 384 well Echo plate | Labcyte | PP-0200 |

### 2. Experimental steps

First, a stock solution of the compound at a concentration of 10 mM was prepared in a test tube with 90% DMSO (10% water), and it was used to prepare a series of dilutions with a dilution factor of 1:5 and final concentrations starting from 100 µM to as low as 0.05 nM.

0.2 µl of the compound solution was transferred into a 384-well reaction plate, and 0.2 µl of 100% DMSO was transferred into both the negative and positive controls. Then, 10 µl of 2× PDE4B1 enzyme solution (making a final concentration of 0.04 nM) was added to each well, and 10 µl of 1× reaction buffer instead of the enzyme solution was added to the zero enzyme activity control wells. The plate was centrifuged at 1,000 rpm for 1 min and incubated at room temperature for 15 min. Next, 10 µl of 2× FAM-cAMP substrate solution (making a substrate final concentration of 0.1 µM) was added to each well of the 384-well reaction plate. The plate was centrifuged at 1,000 rpm for 1 min and the reaction was carried out at 25°C for 30 min. After completion of the reaction, 60 µl of reaction stop solution was added to each well of the 384-well reaction plate to terminate the reaction, and the plate was incubated with shaking at 600 rpm on a shaker at room temperature in the dark for 60 min.

After the incubation, the RLU data were read and the inhibition rate was calculated. The IC₅₀ value was calculated from the concentration-inhibition fitted curve, wherein the maximum value refers to the reading of the DMSO control and the minimum value refers to the reading of the zero enzyme activity control.

The *in vitro* inhibition of PDE4B1 enzyme activity by the Examples of the present disclosure was determined by the above test, and the measured IC₅₀ value was shown in Table 1.

**Table 1**

| No. | PDE4B1 IC₅₀ (nM) | No. | PDE4B1 IC₅₀ (nM) |
|---|---|---|---|
| Compound **1** | 0.32 | Compound **1-1** | 0.11 |
| Compound **1-2** | 0.4 | Compound **2** | 4.5 |
| Compound **3** | 1.1 | Compound **4** | 0.083 |
| Compound **5** | NA | Compound **6** | 0.41 |
| Compound **7** | 0.055 | Compound **8** | 0.22 |
| Compound **9** | 0.25 | Compound **10** | 0.16 |
| Compound **11-1** | 0.15 | Compound **11-2** | 0.81 |
| Compound **12-1** | 0.17 | Compound **12-2** | 3.62 |
| Compound **A** | 0.3 | | |

| | | | |
|---|---|---|---|
| Note: N/A, not detected | | | |

### Test Example 2 Inhibitory effect of compounds on the release of pro-inflammatory cytokines from peripheral blood mononuclear cells (PBMCs)

Frozen PBMCs were thawed and detected by Trypan blue staining for cell viability and number. The thawed PBMCs were washed with RPMI1640 complete medium (RPMI1640 + 10% FBS + 1% PS) and centrifuged, and the supernatant was discarded. The PBMCs were resuspended with RPMI1640 complete medium and the cell density was adjusted to 2×10⁶ cells/mL. 2×10⁵ PBMC cells were plated in a 96-well cell culture plate, and the compounds to be tested were added at different concentrations, in a 1:5 serial dilution of 9 concentrations starting from a maximum compound concentration of 100 µM, each in duplicate. LPS was added at a final concentration of 0.1 ng/mL, making a total volume of 200 µL. For the negative and positive controls, only LPS and DMSO were added to the negative control well, while 1 µg/mL of dexamethasone was added to the positive control well in addition to cells and LPS as. The cells were incubated in an incubator at 37°C for 24 hours. After completion of the incubation, 100 µL of cell culture supernatant was collected and detected by ELISA for the TNF-α level. 100 µL of CellTiter-Glo was added to the remaining cells in each well to detect the cell viability level. The IC₅₀ value of inhibition of TNF-α release by the compounds was calculated.

The *in vitro* inhibition of pro-inflammatory cytokine release of PBMCs by the Examples of the present disclosure was determined by the above test, and the determined IC₅₀ value was shown in Table 2.

**Table 2**

| No. | PBMC IC₅₀ (nM) | No. | PBMC IC₅₀ (nM) |
|---|---|---|---|
| Compound **1-1** | 0.32 | Compound **1-2** | 3.26 |
| Compound **2** | NA | Compound **3** | NA |
| Compound **4** | 2.15 | Compound **5** | 1.56 |
| Compound **6** | 1.56 | Compound **7** | 0.36 |
| Compound **8** | 0.74 | Compound **9** | 0.3 |
| Compound **10** | 0.9 | Compound **11-1** | < 0.025 |
| Compound **12-1** | 0.055 | Compound **A** | 1.16 |

| | | | |
|---|---|---|---|
| Note: N/A, not detected | | | |

### Test Example 3 Experiment of in vitro inhibition of IL-23 secretion of DC cells differentiated from human mononuclear cells by the compounds

Day 0: Mononuclear cells were isolated from fresh human peripheral blood and purified and then resuspended in RPMI-1640 complete medium (RPMI-1640 + 10% FBS + 1% P.S. + 55 µM 2-Mercaptoethanol). When differentiating into DC cells, IL-4 at a concentration of 50 ng/ml and GM-CSF at 100 ng/ml were added to the medium. The cells were cultured at a density of 1×10⁶ cells/mL in a culture dish with a diameter of 100 mm for differentiation in an incubator at 37°C with a carbon dioxide concentration of 5%. Day 3: Half the volume of RPMI complete medium was replaced with fresh RPMI complete medium while maintaining the concentration of IL-4 at 50 ng/ml and GM-CSF at 100 ng/ml. Day 6: Non-adherent cells (DC cells) in the culture dish were collected and washed with PBS. The washed DC cells were resuspended with RPMI complete medium at a density of 1×10⁶ cells/mL. Then, 1×10⁵ DC cells were added to each well of the 96-well cell culture plate and pre-incubated with different dilutions of the compounds to be tested (or DMSO blank negative control at equivalent concentration) for 1 hour, followed by addition of 200 µg/ml of the TLR2 agonist Zymosan to stimulate the DC cells for 24 hours. Day 7: After 24 hours of Zymosan stimulation of DC cells, the supernatant in each well of the 96-well plate was collected and detected by ELISA for the concentration of IL-23.

The inhibition of IL-23 secretion of DC cells differentiated from human mononuclear cells by the compounds of the present disclosure was determined by the above test, and the determined IC₅₀ value was shown in Table 3.

**Table 3**

| No. | PBMC IC₅₀ (nM) | No. | PBMC IC₅₀ (nM) |
|---|---|---|---|
| Compound **1-1** | 0.12 | Compound **12-1** | 0.018 |
| Roflumilast | 12.91 | Compound **A** | 0.61 |

### Test Example 4 Experiment of suppression of imiquimod-induced psoriasis

An appropriate amount of Compound **12-1** was formulated into an ointment with the following components: 0.1% of Compound **12-1,** 9% of hexanediol, 78.8% of white petrolatum, 5% of paraffin, 7% of glyceryl mono- and distearate, and 0.1% of dihydroxybutyl toluene, which were mechanically stirred until an ointment was formed.

### 1) Model establishment and drug administration

7-Week-old Balb/c female mice were used and the hair on their back in an area of 2 cm × 3 cm was shaved the day before the experiment. On Days 1-7 of the experiment, the test compounds were applied on the skin for 6 hours, and then imiquimod (IMQ) ointment (Aldara (5%)) was continuously applied to the back skin of mice for 7 days, so as to establish a mouse model of psoriasis, while the control group was given the same dose of petrolatum ointment. On Days 3, 5 and 7, the severity of skin inflammation was assessed, including measurement of skin thickness, crusting and erythema, scored on a 5-point scale (0-4) respectively. The total score was used to assess the severity of skin inflammation. On Day 7 of the experiment, the spleen was collected and weighed, and the percentage of spleen to body weight was calculated to assess the immunosuppressive effect of the drug.

Normal control group, model control group, low, medium and high dose groups of Compound **12-1** (0.01%, 0.03% and 0.1%), 0.03% Compound **1-1** group, and 0.03% reference Compound **A** group were set up in this experiment.

### 2) Assessment indicators

The total score of the degree of skin inflammation was the clinical score, the indicators of which were relatively subjective, and higher score indicated more serious disease. The degree of increase in skin thickness was an objective assessment indicator, and more increase in thickness indicated more serious disease. The ratio of spleen weight to body weight was an objective assessment indicator, and smaller ratio of spleen weight to body weight indicated stronger immunosuppressive effect of the drug.

### 3) Experimental results

### 3.1) Clinical scores

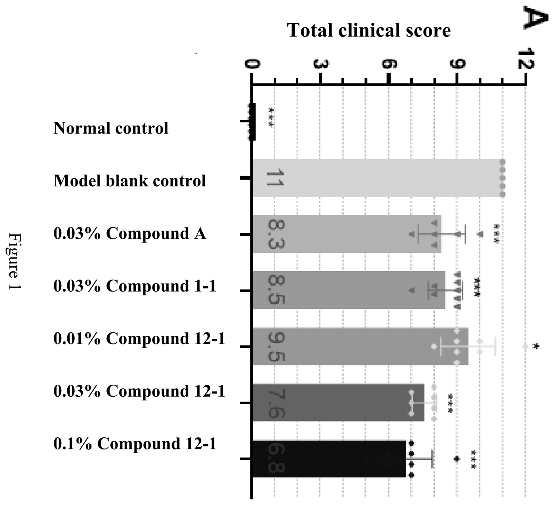

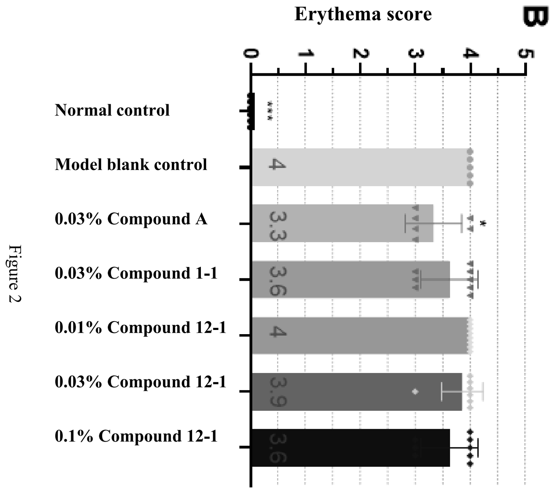

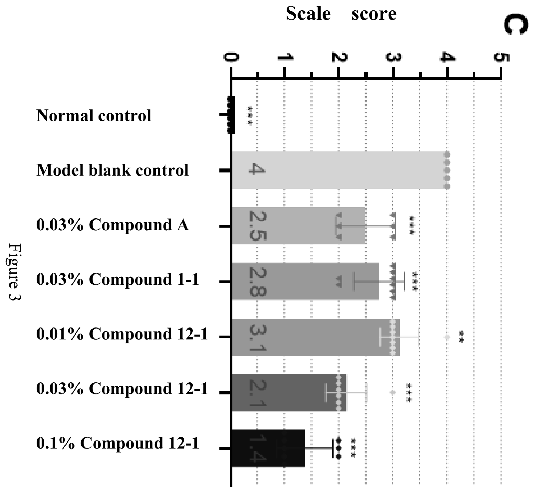

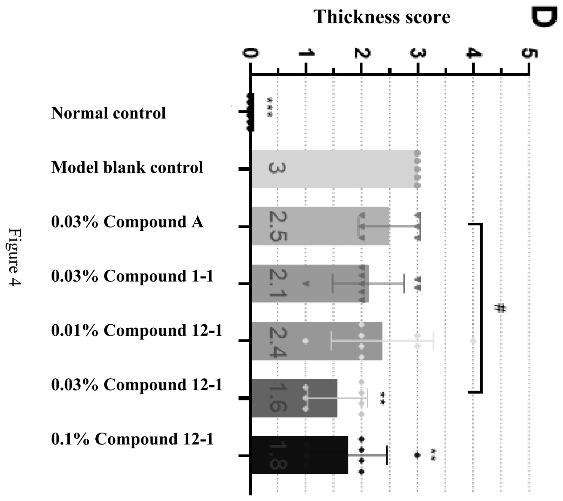

Compound **A,** Compound **1-1** and each dose of Compound **12-1** significantly reduce the clinical score at the endpoint (Figures 1-4). As seen from single item scores, Compound **1-1** and each dose group of Compound **12-1** mainly improve scab and reduce skin thickness in psoriasis model. Among the skin thickness scores, both the low and the high dose groups of Compound **12-1** significantly inhibit the increase of skin thickness, while Compound **A** has no significant effect. 0.03% of Compound **12-1** has significantly higher inhibitory effect on the increase of skin thickness than Compound A at the same dose (Figure 4). The results show that Compound **12-1** is significantly better than the reference Compound **A** in improving psoriasis symptoms. The specific score data are shown in Table 4.

**Table 4**

| Group | Total clinical score | | Erythema score | | Scale score | | Thickness score | |
|---|---|---|---|---|---|---|---|---|
| | Mean | SD | Mean | SD | Mean | SD | Mean | SD |
| Normal control | 0 | 0 | 0 | 0 | 0 | 0 | 0 | 0 |
| Model blank control | 11 | 0 | 4 | 0 | 4 | 0 | 3 | 0 |
| 0.03% Compound **A** | 8.33 | 1.03 | 3.33 | 0.52 | 2.5 | 0.55 | 2.5 | 0.55 |
| 0.01% Compound **12-1** | 9.5 | **1.19** | 4 | 0 | 3.13 | 0.35 | 2.38 | 0.92 |
| 0.03% Compound **12-1** | 7.57 | 0.53 | 3.86 | 0.38 | 2.14 | 0.38 | 1.57 | 0.53 |
| 0.1% Compound **12-1** | 6.75 | 1.17 | 3.63 | 0.52 | 1.38 | 0.52 | 1.75 | 0.71 |
| 0.03% Compound **1-1** | 8.5 | 0.76 | 3.63 | 0.52 | 2.75 | 0.46 | 2.13 | 0.64 |

### 3.2) Ratio of spleen weight to body weight

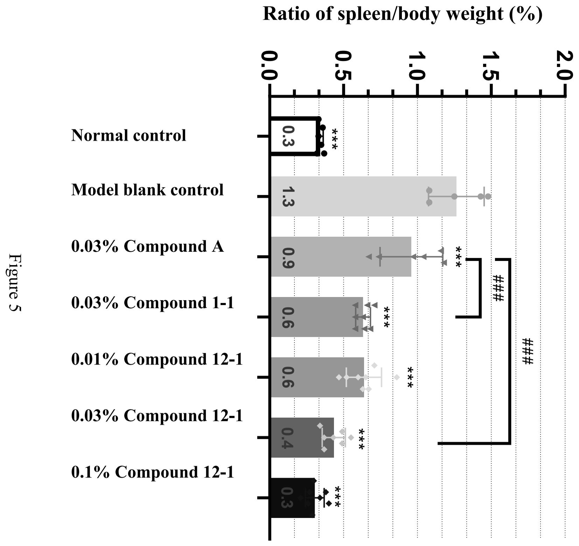

Under the effect of IMQ, there is no significant difference in the body weight of animals in model group and each treatment group, while the ratio of spleen weight to total body weight in model group is increased significantly (Figure 5), indicating splenomegaly. Compound **A** has no significant effect on the ratio of spleen weight to body weight, while Compound **1-1** and the three dose groups of Compound **12-1** significantly reduce the ratio of spleen weight to body weight, indicating that these compounds have immunosuppressive effect, and Compound **12-1** shows a dose-response relationship. At the same dose (0.03%), the effect of Compound **1-1** and Compound **12-1** on the ratio of spleen weight to body weight is significantly different from that of Compound **A,** and these results indicate that the effect of Compound **1-1** and Compound **12-1** on immunity is stronger than that of Compound **A.** The specific data are shown in Table 5.

**Table 5**

| | Normal control | Model blank control | 0.03% Compound **A** | 0.01% Compound **12-1** | 0.03% Compound **12-1** | 0.1% Compound **12-1** | 0.03% Compound **1-1** |
|---|---|---|---|---|---|---|---|
| Mean | 0.34 | 1.26 | 0.96 | 0.64 | 0.43 | 0.31 | 0.63 |
| SD | 0.02 | 0.19 | 0.21 | 0.12 | 0.08 | 0.06 | 0.05 |

## Claims

1. A compound of formula I or a pharmaceutically acceptable salt thereof 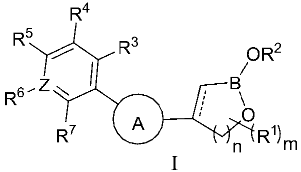
wherein, ring A is selected from the group consisting of 5-6 membered aryl ring and heteroaryl ring, the aryl ring or heteroaryl ring is optionally further substituted with one or more of R^{A1};
R^{A1} is selected from the group consisting of halogen, deuterium, hydroxy, nitro, cyano, amino, C₁₋₆ alkyl, C₃₋₆ cycloalkyl, 3-6 membered heterocyclyl, C₁₋₆ alkoxyl, C₃₋₆ cycloalkoxyl and 3-6 membered heterocycloalkoxyl, the C₁₋₆ alkyl, C₁₋₆ alkoxyl, C₃₋₆ cycloalkoxyl or 3-6 membered heterocycloalkoxyl is optionally further substituted with one or more groups selected from the group consisting of halogen, deuterium, hydroxy, nitro, cyano, amino and C₁₋₆ alkoxyl;
B is a boron atom;
Z is selected from the group consisting of carbon atom and nitrogen atom;
R¹ is each independently selected from the group consisting of hydrogen, halogen, deuterium, hydroxy, oxo, nitro, cyano, amino, C₁₋₆ alkyl, C₃₋₆ cycloalkyl, 3-6 membered heterocyclyl, C₁₋₆ alkoxyl, C₃₋₆ cycloalkoxyl and 3-6 membered heterocycloalkoxyl, the C₁₋₆ alkyl, C₃₋₆ cycloalkyl, 3-6 membered heterocyclyl, C₁₋₆ alkoxyl, C₃₋₆ cycloalkoxyl or 3-6 membered heterocycloalkoxyl is optionally further substituted with one or more of R^{A2};
R^{A2} is selected from the group consisting of halogen, deuterium, hydroxy, nitro, cyano, amino, C₁₋₆ alkyl, C₃₋₆ cycloalkyl, 3-6 membered heterocyclyl, C₁₋₆ alkoxyl, C₃₋₆ cycloalkoxyl and 3-6 membered heterocycloalkoxyl, the C₁₋₆ alkyl, C₁₋₆ alkoxyl, C₃₋₆ cycloalkoxyl or 3-6 membered heterocycloalkoxyl is optionally further substituted with one or more groups selected from the group consisting of halogen, deuterium, hydroxy, nitro, cyano, amino and C₁₋₆ alkoxyl;
R² is selected from the group consisting of hydrogen, alkyl, cycloalkyl, heterocyclyl, aryl and heteroaryl, the alkyl, cycloalkyl, heterocyclyl, aryl or heteroaryl is optionally further substituted with one or more of R^{A3};
R^{A3} is selected from the group consisting of halogen, deuterium, hydroxy, oxo, nitro, cyano and amino;
R³, R⁴ and R⁵ are each independently selected from the group consisting of hydrogen, deuterium, halogen, amino, hydroxy, C₁₋₆ alkyl, C₁₋₆ alkoxyl, C₃₋₆ cycloalkoxyl, C₃₋₆ cycloalkyl, 3-6 membered heterocyclyl and 3-6 membered heterocycloalkoxyl, the alkyl, alkoxyl, cycloalkoxyl, cycloalkyl, heterocyclyl or heterocycloalkoxyl is optionally further substituted with one or more of R^{A4};
R^{A4} is selected from the group consisting of halogen, deuterium, hydroxy, oxo, nitro, cyano and amino;
R⁶ and R⁷, together with the carbon atoms to which they are attached, form a 3-10 membered carbocyclic ring or 3-10 membered heterocyclic ring, the carbocyclic ring or heterocyclic ring is optionally further substituted with one or more of R^{A5};
R^{A5} is selected from the group consisting of halogen, deuterium, hydroxy, oxo, nitro, cyano, C₁₋₆ alkyl, C₁₋₆ alkoxyl, C₃₋₆ cycloalkoxyl, 3-6 membered heterocycloalkoxyl, phenyl and 5-6 membered heteroaryl, the C₁₋₆ alkyl, C₁₋₆ alkoxyl, C₃₋₆ cycloalkoxyl, 3-6 membered heterocycloalkoxyl, C₃₋₈ cycloalkenyloxyl, phenyl or 5-6 membered heteroaryl is optionally further substituted with one or more groups selected from the group consisting of halogen, deuterium, hydroxy, oxo, nitro and cyano;
m is an integer between 0 and 5;
n is an integer between 1 and 3, for example 1 or 2;
and 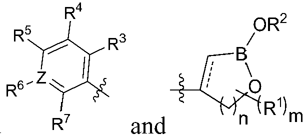 are on the meta-position of ring A;
"/" is a single bond or absent,
and preferably wherein R³ and R⁴ are each independently selected from hydrogen; R⁵ is selected from the group consisting of C₁₋₆ alkyl and C₁₋₆ alkoxyl, the alkyl or alkoxyl is optionally substituted with 1 to 3 R^{A4}, R^{A4} is as defined in claim 1.

2. The compound or a pharmaceutically acceptable salt thereof according to claim 1, wherein the compound of formula I is 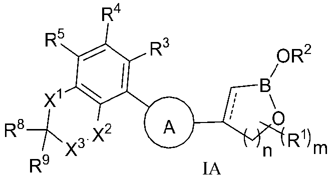
wherein, X¹ is selected from the group consisting of -O-, -N(R^{16a})- and -CR^{16a}R^{16b}-;
X² is selected from the group consisting of -O- and -CR^{17a}R^{17b}-;
X³ is selected from the group consisting of a bond, -CR^{18a}R^{18b}- and - CR^{18a}R^{18b}CR^{18c}R^{18d}-;
R^{16a} and R^{16b} are each independently selected from the group consisting of hydrogen, deuterium, halogen, amino, hydroxy, C₁₋₆ alkyl and C₁₋₆ alkoxyl, the alkyl or alkoxyl is optionally substituted with halogen, nitro, cyano or C₁₋₆ alkoxyl;
R^{17a} and R^{17b} are each independently selected from the group consisting of hydrogen, deuterium, halogen, amino, hydroxy, C₁₋₆ alkyl and C₁₋₆ alkoxyl, the alkyl or alkoxyl is optionally substituted with halogen, nitro, cyano or C₁₋₆ alkoxyl;
R^{18a}, R^{18b}, R^{18c} and R^{18d} are each independently selected from the group consisting of hydrogen, deuterium, halogen, amino, hydroxy, C₁₋₆ alkyl and C₁₋₆ alkoxyl, the alkyl or alkoxyl is optionally substituted with halogen, nitro, cyano or C₁₋₆ alkoxyl;
R⁸ and R⁹ are each independently selected from the group consisting of hydrogen, deuterium, halogen, amino, hydroxy, C₁₋₆ alkyl, C₁₋₆ alkoxyl, C₃₋₆ cycloalkoxyl, C₃₋₆ cycloalkyl, 3-6 membered heterocyclyl and 3-6 membered heterocycloalkoxyl, the alkyl, alkoxyl, cycloalkoxyl, cycloalkyl or heterocyclyl is optionally further substituted with one or more of R^{A6};
or R⁸ and R⁹, together with the carbon atoms to which they are attached, form a 3-6 membered carbocyclic ring or 3-6 membered heterocyclic ring, the carbocyclic ring or heterocyclic ring is optionally further substituted with one or more of R^{A6};
or R⁸ and R⁹ form oxo (=O);
R^{A6} is selected from the group consisting of halogen, deuterium, hydroxy, oxo, nitro, cyano, amino, C₁₋₆ alkyl and C₁₋₆ alkoxyl;
ring A, R¹~R⁵, B, m, n and " " are as defined in claim 1,
and preferably wherein the compound of formula I is

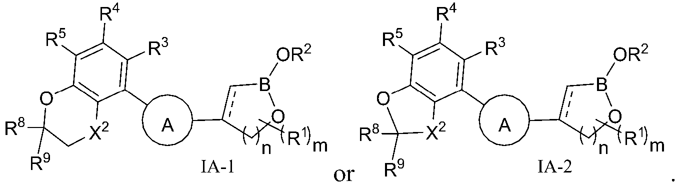

3. The compound or a pharmaceutically acceptable salt thereof according to claim 2, wherein the 3-6 membered carbocyclic ring or 3-6 membered heterocyclic ring formed by R⁸ and R⁹ with the carbon atoms to which they are attached, is selected from the group consisting of 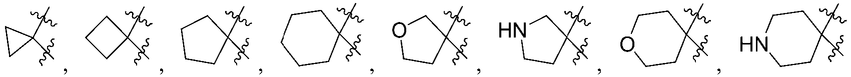 and 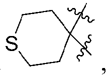 further, the carbocyclic ring or heterocyclic ring is optionally substituted with 1 to 3 R^{A6}; R^{A6} is as defined in claim 2.

4. The compound or a pharmaceutically acceptable salt thereof according to claim 1, wherein the compound of formula I is 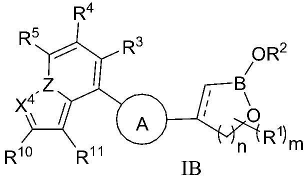
wherein, X⁴ is selected from the group consisting of nitrogen atom and carbon atom;
R¹⁰ and R¹¹ are each independently selected from the group consisting of hydrogen, deuterium, halogen, amino, hydroxy, C₁₋₆ alkyl, C₁₋₆ alkoxyl, C₃₋₆ cycloalkoxyl, C₃₋₆ cycloalkyl, 3-6 membered heterocyclyl and 3-6 membered heterocycloalkoxyl, the alkyl, alkoxyl, cycloalkoxyl, cycloalkyl or heterocyclyl is optionally further substituted with one or more of R^{A7};
R^{A7} is selected from the group consisting of halogen, deuterium, hydroxy, oxo, nitro, cyano, amino, C₁₋₆ alkyl and C₁₋₆ alkoxyl;
ring A, Z, R¹~R⁵, B, m, n and " " are as defined in claim 1.

5. The compound or a pharmaceutically acceptable salt thereof according to claim 1, wherein the compound of formula I is 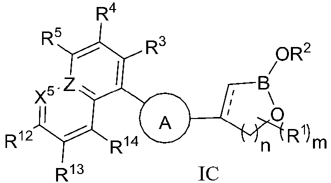
wherein, X⁵ is selected from the group consisting of nitrogen atom and carbon atom;
R¹², R¹³ and R¹⁴ are each independently selected from the group consisting of hydrogen, deuterium, halogen, amino, hydroxy, C₁₋₆ alkyl, C₁₋₆ alkoxyl, C₃₋₆ cycloalkoxyl, C₃₋₆ cycloalkyl, 3-6 membered heterocyclyl and 3-6 membered heterocycloalkoxyl, the alkyl, alkoxyl, cycloalkoxyl, cycloalkyl or heterocyclyl is optionally further substituted with one or more of R^{A8};
R^{A8} is selected from the group consisting of halogen, deuterium, hydroxy, oxo, nitro, cyano, amino, C₁₋₆ alkyl and C₁₋₆ alkoxyl;
ring A, Z, R¹~R⁵, B, m, n and " " are as defined in claim 1.

6. The compound or a pharmaceutically acceptable salt thereof according to claim 5,
wherein R¹², R¹³ and R¹⁴ are each independently selected from the group consisting of hydrogen, deuterium, halogen, amino, hydroxy, C₁₋₆ alkyl and C₁₋₆ alkoxyl, the alkyl or alkoxyl is optionally substituted with 1 to 3 R^{A8}; R^{A8} is as defined in claim 5,
wherein R^{A8} is selected from the group consisting of halogen, C₁₋₆ alkyl and C₁₋₆ alkoxyl, preferably fluorine, chlorine, methyl, ethyl, methoxyl or ethoxyl, and
wherein X⁵ is selected from nitrogen atom; Z is selected from carbon atom.

7. The compound or a pharmaceutically acceptable salt thereof according to any one of claims 1-6, wherein ring A is selected from the group consisting of: 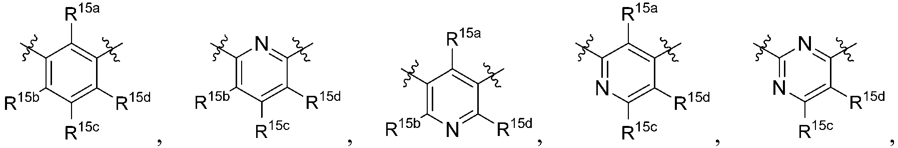 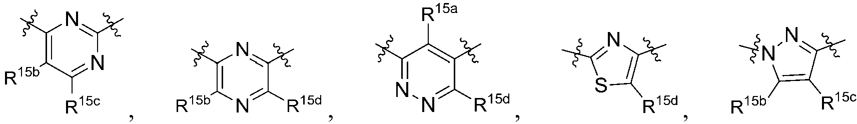 and 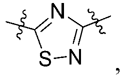 wherein R^{15a}, R^{15b}, R^{15c} and R^{15d} are each independently selected from the group consisting of hydrogen, deuterium, halogen, amino, hydroxy, C₁₋₆ alkyl and C₁₋₆ alkoxyl, the alkyl or alkoxyl is optionally further substituted with one or more groups selected from the group consisting of halogen, deuterium, hydroxy, nitro, cyano and amino, further, ring A is preferably 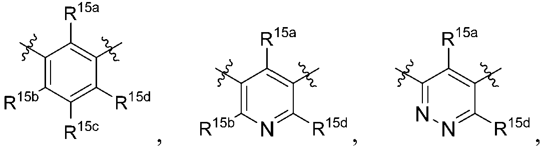 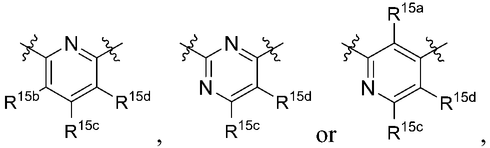 and preferably wherein R^{15a}, R^{15b}, R^{15c} and R^{15d} are each independently selected from the group consisting of hydrogen, deuterium, halogen and C₁₋₆ alkyl, preferably hydrogen, fluorine, chlorine, methyl or ethyl.

8. The compound or a pharmaceutically acceptable salt thereof according to any one of claims 1-7,
wherein n is selected from 1 and 2,
wherein R² is selected from the group consisting of hydrogen and C₁₋₆ alkyl, preferably hydrogen, methyl or ethyl, more preferably hydrogen, and
wherein R¹ is each independently selected from the group consisting of hydrogen, deuterium, halogen, amino, hydroxy, C₁₋₆ alkyl and C₁₋₆ alkoxyl, the alkyl or alkoxyl is optionally further substituted with one or more groups selected from the group consisting of halogen, deuterium, hydroxy, nitro, cyano and amino, preferably R¹ is each independently selected from the group consisting of hydrogen, fluorine, chlorine, methyl, ethyl, methoxyl or ethoxyl.

9. The compound or a pharmaceutically acceptable salt thereof according to any one of claims 1-8, wherein the compound of formula I is selected from the group consisting of: 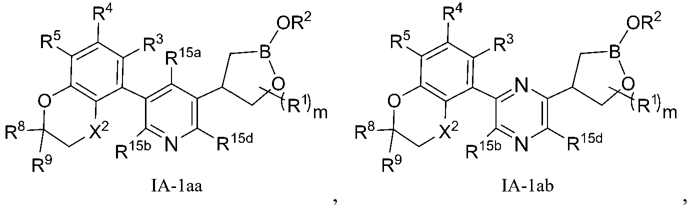 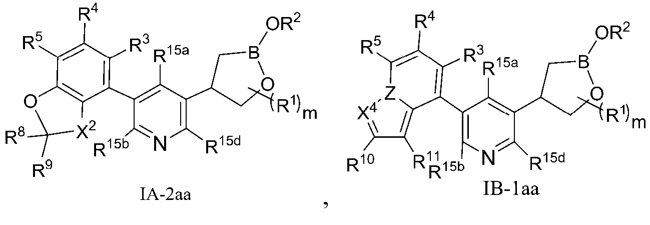 and 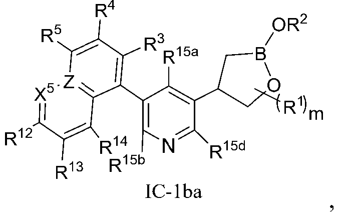 wherein, R¹~R⁵, B and m are as defined in claim 1, R⁸ and R⁹ are as defined in claim 2, R^{15a}, R^{15b} and R^{15d} are as defined in claim 7, further, the compound of formula I is preferably 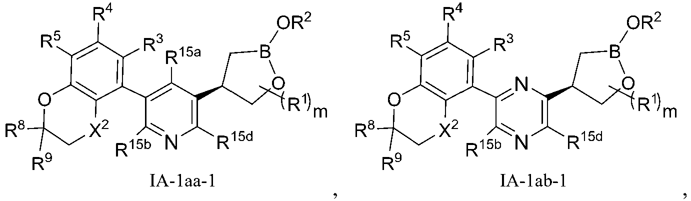 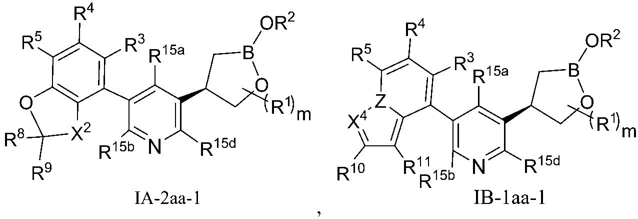 or

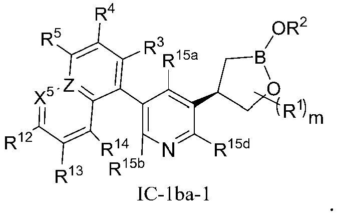

10. The compound of formula I or a pharmaceutically acceptable salt thereof according to any one of claims 1-9, wherein the compound of formula I is selected from the group consisting of: 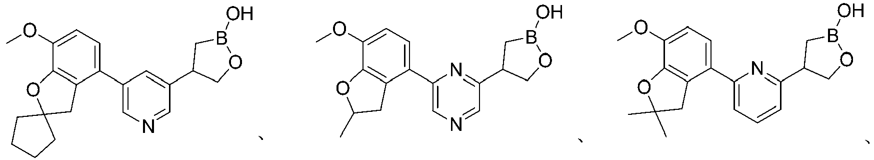 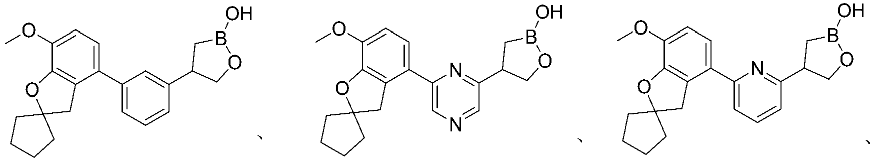 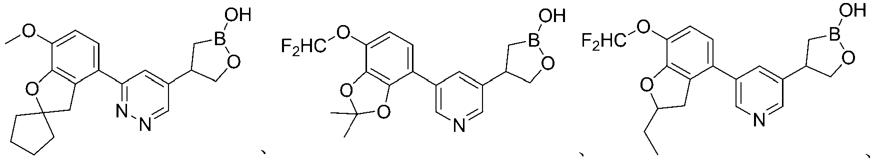 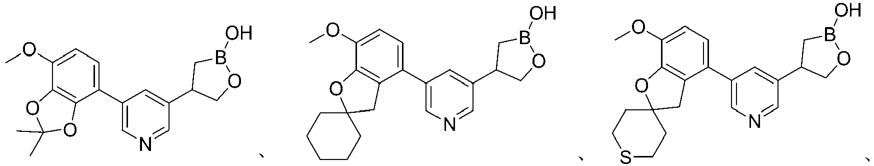 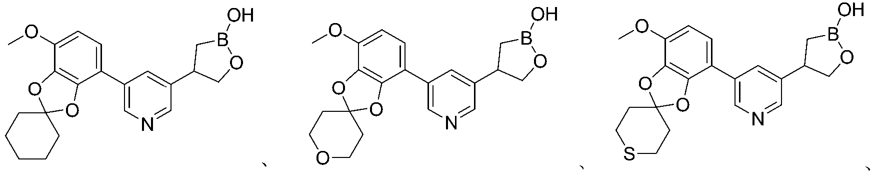 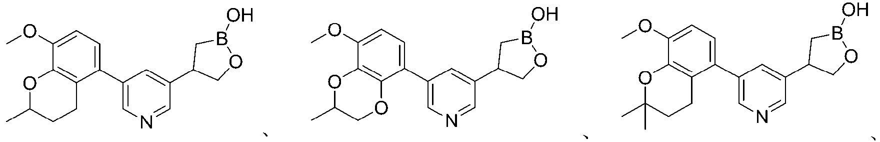 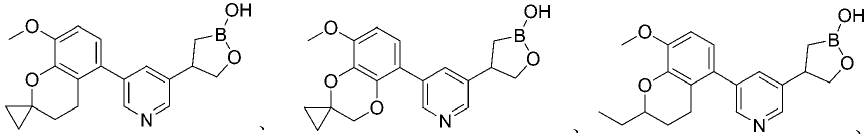 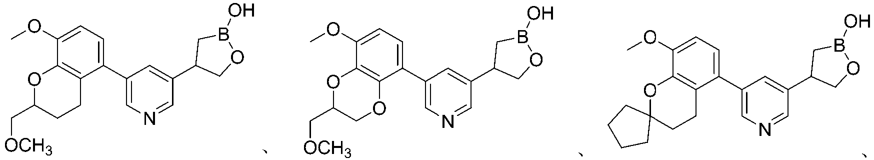 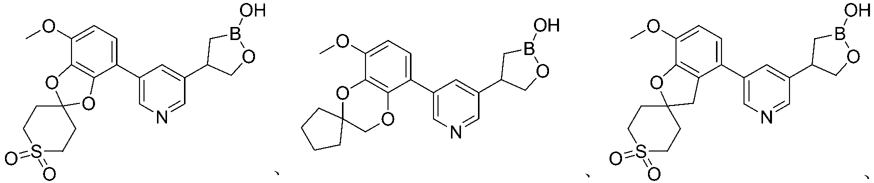 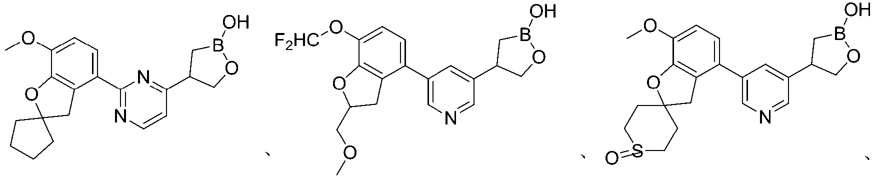 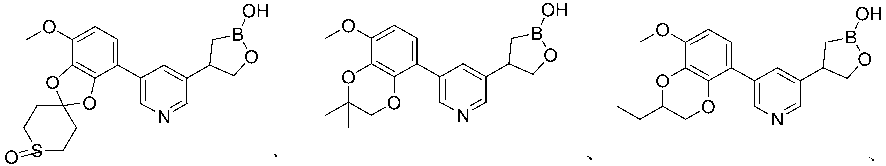 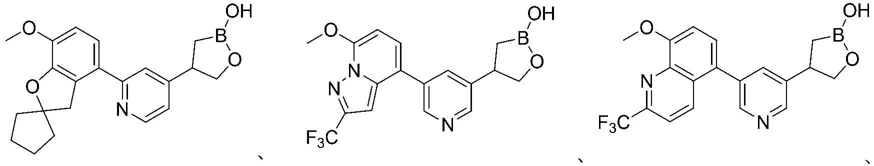 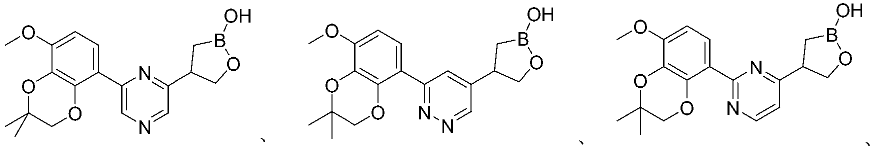 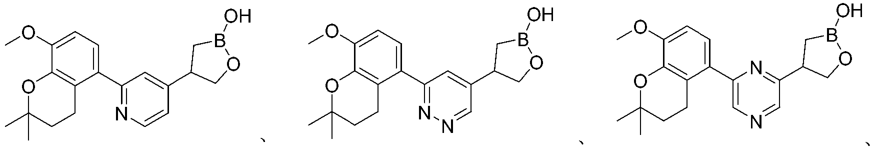 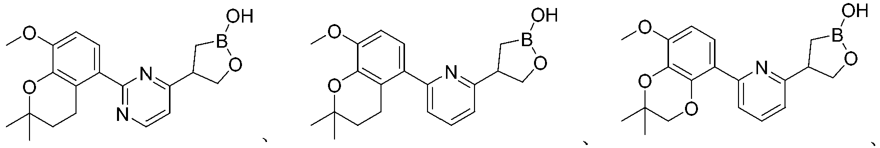 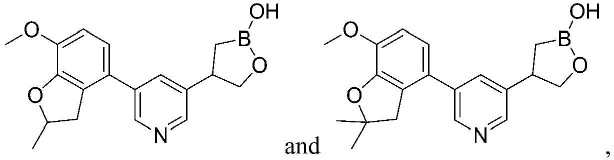 further, the compound of formula I is preferably selected from the group consisting of:

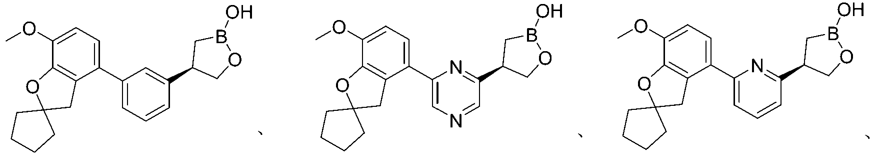

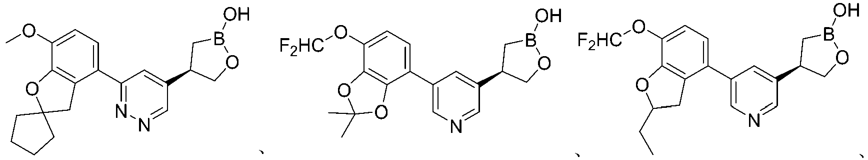

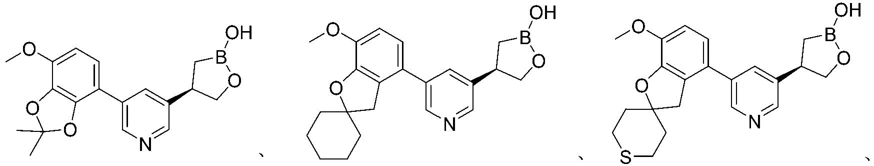

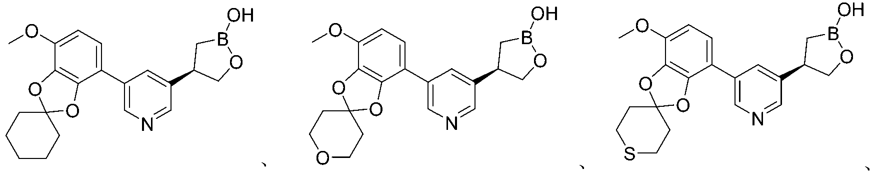

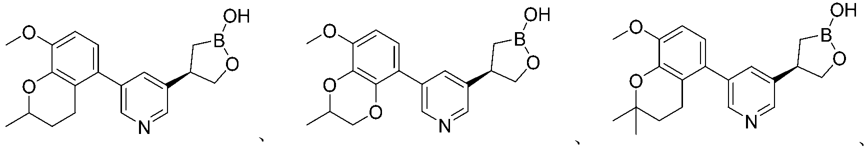

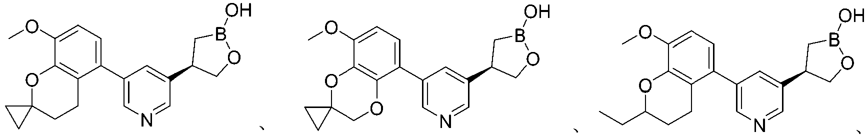

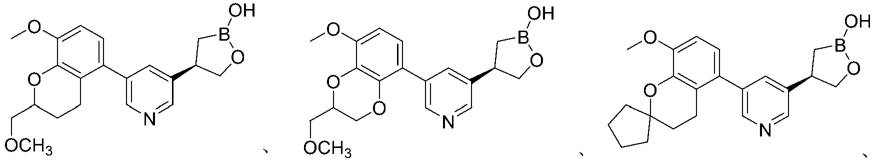

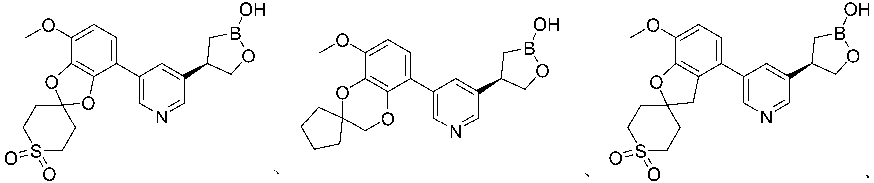

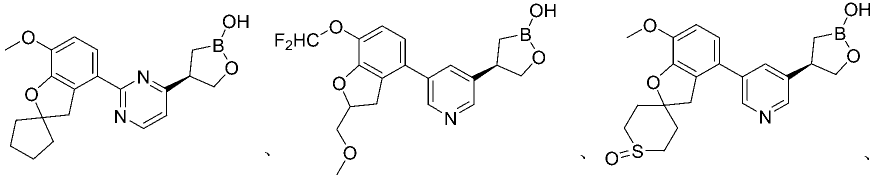

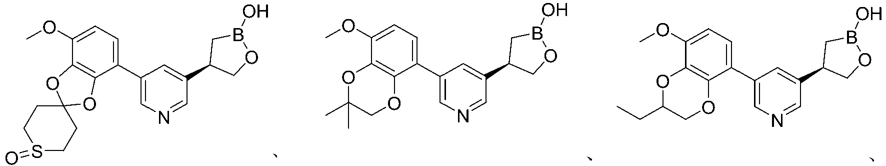

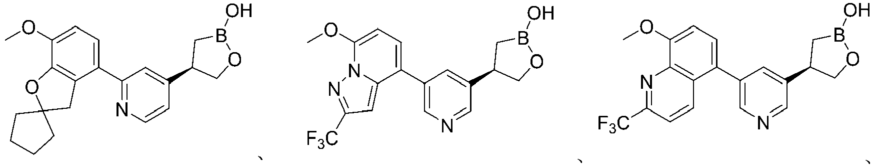

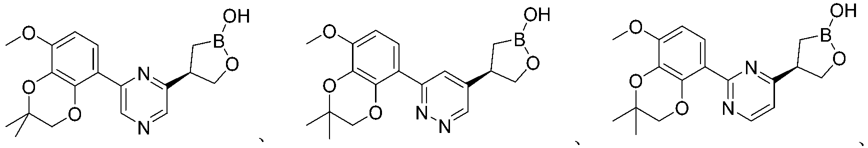

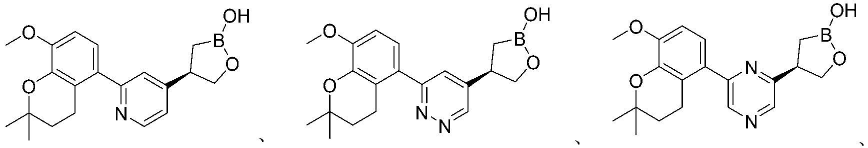

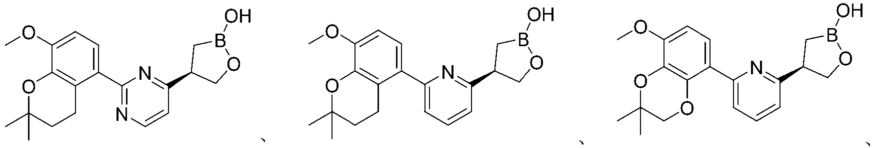

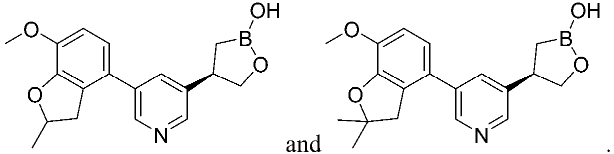

11. Isotopic substituted compound of the compound or a pharmaceutically acceptable salt thereof according to any one of claims 1-10, preferably, the isotopic substituted compound is a deuterated compound.

12. A compound selected from the group consisting of:

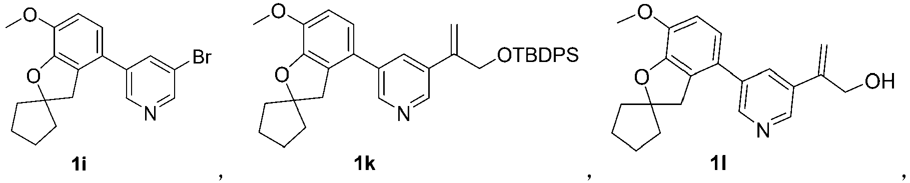

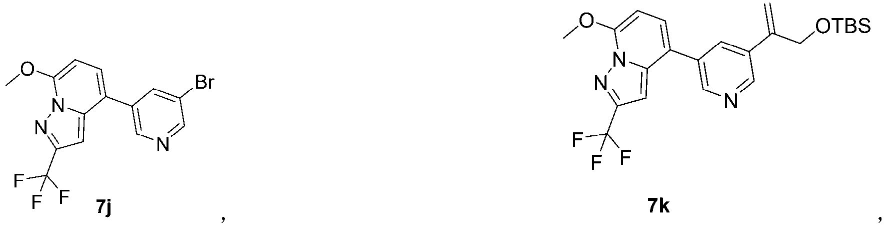

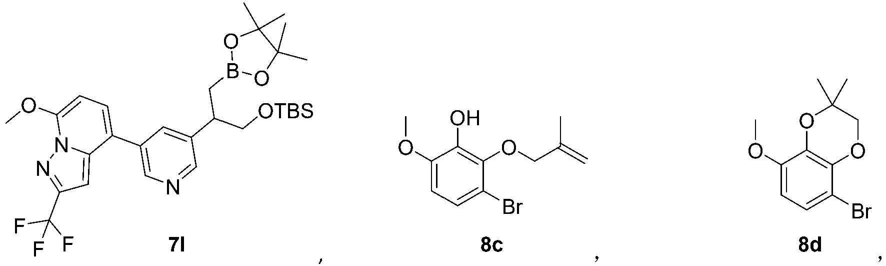

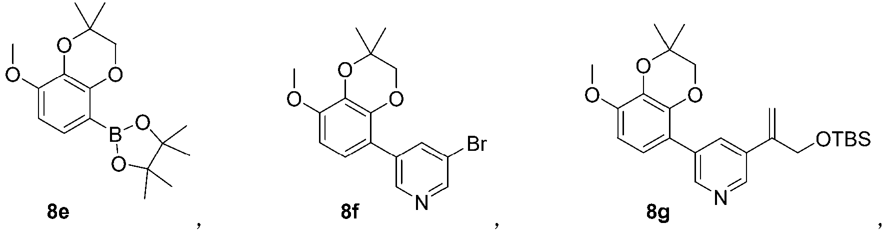

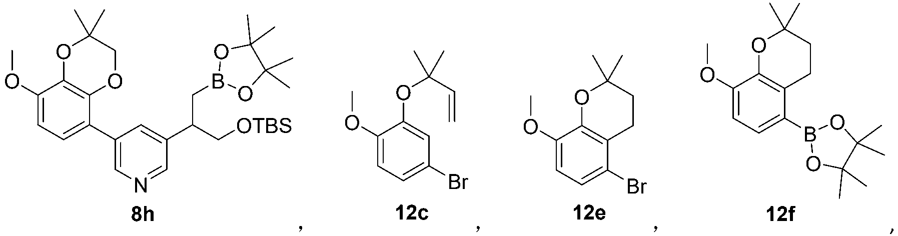

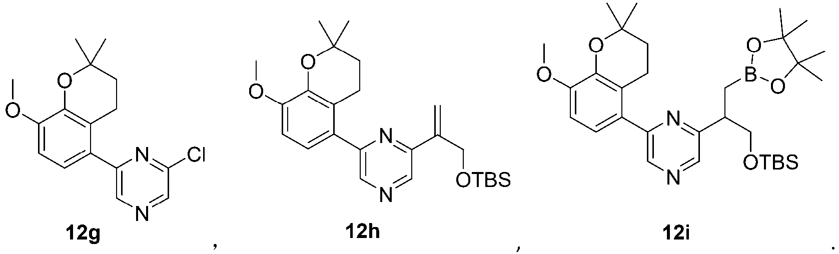

13. A pharmaceutical composition comprising a therapeutically effective amount of at least one of the compound or a pharmaceutically acceptable salt thereof according to any one of claims 1-10, or the isotopic substituted compound according to claim 11, as well as a pharmaceutically acceptable excipient.

14. The compound according to any one of claims 1-10, or the isotopic substituted compound according to claim 11, or the pharmaceutical composition according to claim 13 for use in the treatment of:
- a PDE-related disorder, or
- of asthma, obstructive pulmonary disease, septicaemia, nephritis, diabetes, allergic rhinitis, allergic conjunctivitis, ulcerative enteritis, rheumatism or psoriasis.
